# EUROPEAN PATENT SPECIFICATION

(11) **EP 4 166 101 B1**
(45) Date of publication and mention of the grant of the patent: **10.09.2025**
(21) Application number: 21821051.6
(22) Date of filing: 28.05.2021
(51) Int. Cl.: A61H 23/02, G16H 20/17, G16H 20/30, G16H 40/63, A61H 39/00, A61H 1/00, A61N 1/36

(54) **TREATMENT PEN, MAIN MACHINE, AND LIMB PAIN TREATMENT INSTRUMENT**
BEHANDLUNGSSTIFT, HAUPTMASCHINE UND GLIEDMASSENSCHMERZBEHANDLUNGSINSTRUMENT
STYLO DE TRAITEMENT, MACHINE PRINCIPALE ET INSTRUMENT DE TRAITEMENT DE LA DOULEUR DES MEMBRES

(30) Priority: 11.06.2020 CN 202010527370; 11.06.2020 CN 202021064963 U; 11.06.2020 CN 202021064456 U; 11.06.2020 CN 202021065001 U; 08.01.2021 CN 202110025049; 08.01.2021 CN 202110023699; 08.01.2021 CN 202110024835; 08.01.2021 CN 202120044429 U; 08.01.2021 CN 202110022913; 08.01.2021 CN 202110022888; 08.01.2021 CN 202120043354 U; 08.01.2021 CN 202110021753; 08.01.2021 CN 202110021255
(43) Date of publication of application: 19.04.2023
(73) Proprietor: Yunnan Liheng Medical Technology Co., Ltd., Kunming, Yunnan 650200 (CN)
(72) Inventor: WANG, Xing, Kunming, Yunnan 650200 (CN); LI, Kaisheng, Kunming, Yunnan 650200 (CN); CHEN, Yuan, Kunming, Yunnan 650200 (CN); ZHOU, Jigang, Kunming, Yunnan 650200 (CN)
(74) Representative: IPAZ
(86) International application number: PCT/CN2021/096836
(87) International publication number: WO 2021/249211

(56) References cited:
- WO-A1-2019/053469
- CN-A- 1 084 773
- CN-A- 111 671 645
- CN-U- 2 090 239
- CN-U- 204 890 871
- DE-A1- 10 042 438
- KR-A- 20200 059 845
- TW-B- 510 797
- US-A1- 2015 073 357

## Description

The present application claims the priorities to a patent application No. 202010527370.6 filed with the China National Intellectual Property Administration on June 11, 2020 and entitled "LIMB PAIN TREATMENT PEN UTILIZING PHYSICAL KINETIC ENERGY AND TREATMENT INSTRUMENT APPLYING SAME", an utility model No. 202021064963.5 filed with the China National Intellectual Property Administration on June 11, 2020 and entitled "TREATMENT PEN WITH ULTRAVIOLET STERILIZATION FUNCTION", an utility model No. 202021064456.1 filed with the China National Intellectual Property Administration on June 11, 2020 and entitled "TREATMENT PEN BASED ON ELECTROMAGNETIC VIBRATION", an utility model 202021065001.1 filed with the China National Intellectual Property Administration on June 11, 2020 and entitled "TREATMENT INSTRUMENT FOR LIMB PAIN", a patent application No. 202110021753.0 filed with the China National Intellectual Property Administration on January 08, 2021 and entitled "PULSE MODULATION SYSTEM, PRESCRIPTION GENERATION METHOD, APPARATUS, AND DEVICE", a patent application No. 202110021255.6 filed with the China National Intellectual Property Administration on January 08, 2021 and entitled "METHOD FOR GENERATING PRESCRIPTION, DEVICE AND EQUIPMENT", a patent application No. 202110025049.2 filed with the China National Intellectual Property Administration on January 08, 2021 and entitled "TREATMENT INSTRUMENT FOR LIMB PAIN", a patent application No. 202110023699.3 filed with the China National Intellectual Property Administration on January 08, 2021 and entitled "TREATMENT PEN OF LIMB PAIN TREATMENT INSTRUMENT", a patent application No. 202110024835.0 filed with the China National Intellectual Property Administration on January 08, 2021 and entitled "TREATMENT PEN OF LIMB PAIN TREATMENT INSTRUMENT", an utility model No. 202120044429.6 filed with the China National Intellectual Property Administration on January 08, 2021 and entitled "MAIN MACHINE OF LIMB PAIN TREATMENT INSTRUMENT", a patent application No. 202110022913.3 filed with the China National Intellectual Property Administration on January 08, 2021 and entitled "TREATMENT PEN OF LIMB PAIN TREATMENT INSTRUMENT", a patent application No. 202110022888.9 filed with the China National Intellectual Property Administration on January 08, 2021 and entitled "TREATMENT PEN OF LIMB PAIN TREATMENT INSTRUMENT", an utility model No. 202120043354.X filed with the China National Intellectual Property Administration on January 08, 2021 and entitled "VIBRATION TESTING DEVICE FOR TREATMENT PEN OF LIMB PAIN TREATMENT INSTRUMENT".

### Technical field

The invention relates to the technical field of medical equipments, in particular to a treatment pen, a main machine and a limb pain treatment instrument.

### Background

Data shows that, the prevalence of chronic pain is about 38% in the world, with the prevalence of 37% in developed countries and 41% in developing countries.

The applicant finds that when the muscles, tendons and other soft tissues of the limbs are suffered from local closed injuries, spasms, edema, adhesions and other phenomena would occur and lesions would be formed. Since there are abundant and sensitive receptors (including pain receptors and other receptors) in the skin and other tissues of the human body, the receptors at the lesions will suffer from pain and other symptoms due to stimulation.

After the formation of the above lesions, the human body will show different degrees of clinical manifestations such as pain, deformity, dyskinesia, etc., which will cause long-term troubles and injuries to the life and quality of life of patients and their families. However, the existing treatment methods, such as traditional surgery, minimally invasive surgery, shock wave, acupuncture, massage, cupping and radiofrequency ablation, etc., may only achieve temporary relief or treatment with potential safety risks.

Therefore, in order to remove the lesion safely, quickly, efficiently, and even completely, higher and more specific requirements are put forward for the safety, timeliness, accuracy, intensity and other functions of the treatment technology and equipment.

KR20200059845A discloses a needle vibration stimulator. The needle vibration stimulator comprises a needle body mounting part which surrounds at least a part of an outer circumference of a needle body and made of a magnet such that one end of the needle body forming a needle is inserted and combined therein in a longitudinal direction of the needle body; and a motor part which applies vibrations to the needle body mounting part. According to the present invention, the needle body can be firmly gripped by the needle body mounting part.

DE10042438A1 discloses a device for the treatment of predetermined acupuncture points, namely without needle stitches as in traditional acupuncture and also without electrical treatment, as both types of treatment are often rejected by the group of people in need. DE10042438A1 further discloses a motor-driven pen which, when placed on the acupuncture points, applies a two-dimensional stimulus and a three-dimensional stimulus with an adapter.

### Summary

Embodiments of the present disclosure provide a treatment pen for a limb pain treatment instrument including a main machine that outputs a vibration prescription signal to the treatment pen, so as to realize the treatment of lesion sites of spasm, edema, adhesion and nodules in the soft tissues (such as muscles, tendons, etc.) of the limbs due to the local closed damage by using the limb pain treatment instrument, thereby achieving the purpose of improving pain and even eliminating the lesions.

The invention is set out in the appended set of claims.

The limb pain treatment instrument provided by the embodiments of the present disclosure comprises the treatment pen and the main machine, a touch display screen is arranged on the surface of the chassis of the main machine, so that a user may select a vibration prescription via the touch display screen. The main control board of CPU may output the vibration prescription signal corresponding to the vibration prescription to the vibrator of the treatment pen. The vibrator can generate physical kinetic energy corresponding to the vibration prescription signal when it is started and receives the vibration prescription signal, and transmit the physical kinetic energy to the treatment needle installed at the front end of the pen rod. When the treatment needle intervenes into the lesion site for treatment, the physical kinetic energy is intervened into the lesion site with the treatment needle, so that the lesions such as the damaged adhesions and nodules may be better loosened to realize the treatment of the lesion site, and thus achieve the purpose of improving pain or even eliminating the lesion.

Of course, it is not necessary to achieve all the advantages mentioned above at the same time to implement the products or methods of any embodiment of the present disclosure.

### Brief Description of the Drawings

In order to explain the technical solutions of the embodiments and the prior art of the present disclosure more clearly, the following is a brief introduction of the accompanying drawings needed the embodiments and the prior art.
Fig. 1 is a schematic structural diagram of a pulse modulation system according to some embodiments of the present disclosure;
Fig. 2 is a schematic structural diagram of a control device according to some embodiments of the present disclosure;
Fig. 3 is a schematic flow diagram of pulse modulation by a control device according to some embodiments of the present disclosure;
Fig. 4 is a schematic waveform diagram of various pulse waveform structures in some embodiments of the present disclosure;
Fig. 5 is a schematic waveform diagram of a preset pulse in some embodiments of the present disclosure;
Fig. 6 is a schematic structural diagram of a pulse modulation apparatus according to some embodiments of the present disclosure;
Fig. 7 is a schematic structural diagram of an electronic equipment according to some embodiments of the present disclosure;
Fig. 8 is a schematic structural diagram of a limb pain treatment instrument according to some embodiments of the present disclosure;
Fig. 9 is a front view of a treatment pen of a limb pain treatment instrument according to some embodiments of the present disclosure;
Fig. 10 is a first explosive structure diagram of a treatment pen of a limb pain treatment instrument according to some embodiments of the present disclosure;
Fig. 11 is a front view of a main machine of the limb pain treatment instrument according to some embodiments of the present disclosure;
Fig. 12 is a schematic structural diagram of a CPU main control board of the main machine according to some embodiments of the present disclosure;
Fig. 13 is a perspective view of the main machine of the limb pain treatment instrument according to some embodiments of the present disclosure;
Fig. 14 is a cross-sectional view of the treatment pen shown in Fig. 9 taken along the A-A direction;
Fig. 15 is a schematic diagram of traces of the vibration of a vibrator according to some embodiments of the present disclosure;
Fig. 16 is another structural schematic diagram of the treatment pen of the limb pain treatment instrument according to some embodiments of the present disclosure;
Fig. 17 is a second explosive structure diagram of the treatment pen of the limb pain treatment instrument according to some embodiments of the present disclosure;
Fig. 18 is a schematic structural diagram of the limb pain treatment instrument from another perspective according to some embodiments of the present disclosure;
Fig. 19 is a schematic structural diagram of a treatment needle according to some embodiments of the present disclosure;
Fig. 20 is a schematic diagram of a PCB control board of the treatment pen of the limb pain treatment instrument according to some embodiments of the present disclosure;
Fig. 21 is a schematic structural diagram of a rotary centrifugal vibrator in some embodiments of the present disclosure;
Fig. 22 is a schematic diagram of a vibration testing device according to some embodiments of the present disclosure;
Fig. 23a is a front view of a bracket in some embodiments of the present disclosure;
Fig. 23b is a left side view of a bracket in some embodiments of the present disclosure;
Fig. 23c is a top view of a bracket in some embodiments of the present disclosure;
Fig. 23d is a cross-sectional view of Fig. 23a taken along the B-B direction;
Fig. 24 is a schematic diagram of a support lug in some embodiments of the present disclosure;
Fig. 25 is a schematic diagram of a support lug in other embodiments of the present disclosure;
Fig. 26 is a top view of a bracket in other embodiments of the present disclosure;
Fig. 27 is a top view of a bracket in still other embodiments of the present disclosure;
Fig. 28 is a top view of a treatment pen placed on two brackets according to some embodiments of the present disclosure;
Fig. 29 is a top view of a treatment pen placed on two brackets according to other embodiments of the present disclosure;
Fig. 30 is a top view of a treatment pen placed on two brackets according to still other embodiments of the present disclosure.

### Detailed Description

In order to make the purpose, technical solution and advantages of the present disclosure clearer, the present disclosure will be further explained in detail hereinafter with reference to the accompanying drawings and embodiments.

Embodiments of the present disclosure provide a pulse modulation system, method, apparatus and device. The method and apparatus can be applied to various electronic devices, but are not limited in detail. Firstly, the pulse modulation system provided by an embodiment of the present disclosure will be described in detail.

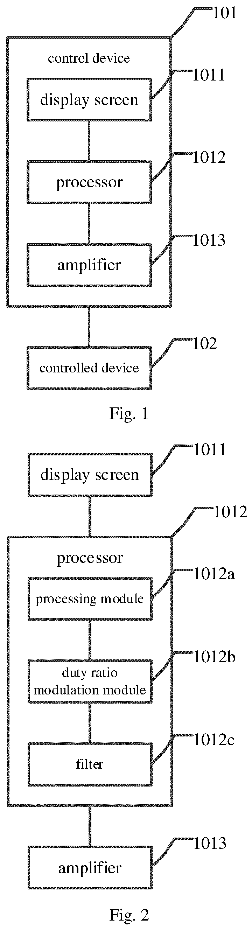

Fig. 1 is a schematic structural diagram of a pulse modulation system provided by an embodiment of the present disclosure, which includes a control device 101 and a controlled device 102, wherein the control device 101 includes a display screen 1011, a processor 1012 and an amplifier 1013, and the processor 1012 is connected to the display screen 1011 and the amplifier 1013 respectively;
the display screen 1011 is configured for displaying identifications of a plurality of pulse waveforms in the visual interface, and receiving the user's selection instruction for the identifications of pulse waveforms.

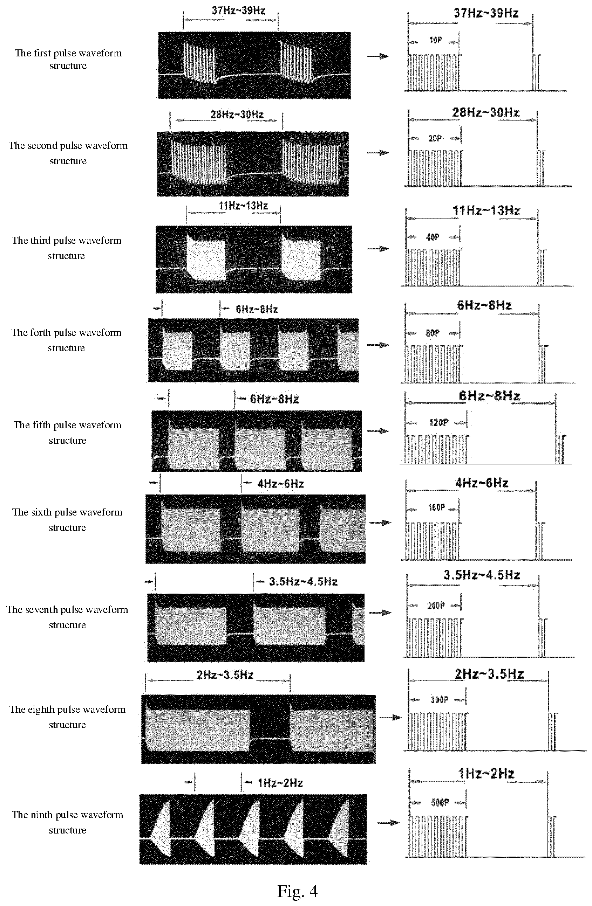

The type of the display screen 1011 may be a touch display screen, a key display screen, and the like, and the specific type of the display screen is not limited. Take the display screen 1011 is a touch display screen as an example, the identifications of various pulse waveforms may be displayed in the visual interface. The identifications may be names corresponding to various pulse waveforms, schematic waveform diagrams corresponding to various pulse waveforms, etc., and the specific identifications are not limited. The names corresponding to pulse waveforms may be "Physical Prescription I", "Physical Prescription II" and so on, and the names corresponding to specific pulse waveforms are not limited. After the user touches the display screen, the user's selection instruction for the identifications of the pulse waveforms may be determined according to the position of the user's touch point on the display screen. The various pulse waveforms corresponding to the identifications of the various pulse waveforms displayed in the visual interface may be referred to Fig. 4 for details, Fig. 4 is a waveform schematic diagram of various pulse waveform structures provided by the embodiments of the present disclosure. The selection instruction is an instruction for selecting an identification of a pulse waveform, which may also be an instruction for selecting for selecting a pulse waveform. Since the pulse waveform contains information such as pulse frequency and pulse intensity, the selection instruction may also be understood as an instruction for selecting information such as pulse frequency and pulse intensity.

The processor 1012, is configured for determine a modulation mode corresponding to the selection instruction among a plurality of preset modulation modes as a target modulation mode; modulating a preset pulse based on the target modulation mode to obtain a modulated pulse, wherein the waveform of the modulated pulse is consistent with the pulse waveform corresponding to the selection instruction; pulse width duty ratio of the preset pulse is between 10% and 99%, and the pulse frequency is between 0.5KHz and 3KHz; inputting the modulated pulse to the amplifier 1013. Taking example 1 as an example to illustrate an alternative embodiment in the following.

### Example 1

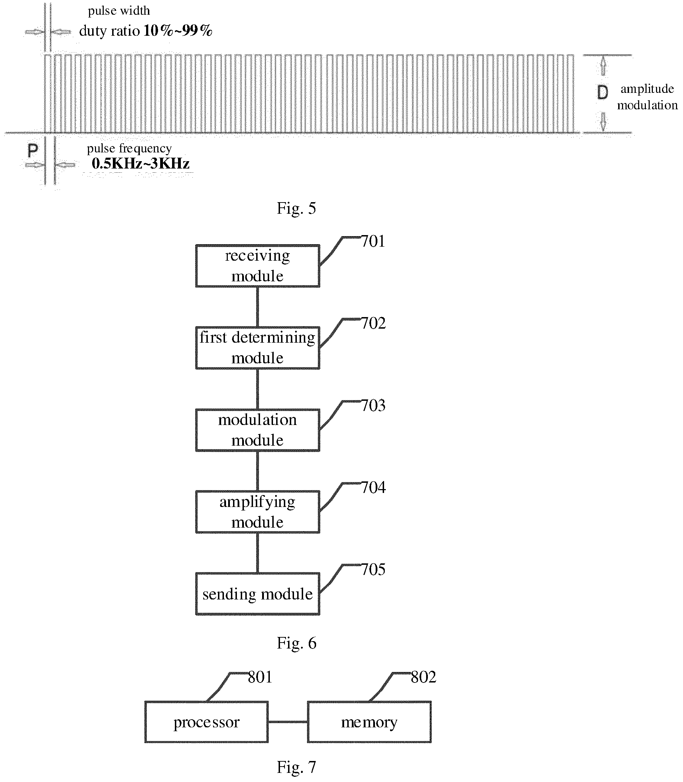

A variety of modulation modes may be preset, each modulation mode corresponds to one pulse waveform, and the pulse waveform contains multiple modulation parameters, such as frequency, intensity, etc., and the specific modulation parameters are not limited. The basic frequency is 1-40Hz, a higher frequency can be modulated on this basis. The above preset pulse is a reference pulse, the preset pulse may be modulated according to the modulation modes corresponding to the selection instruction. In Example 4, the waveform of the preset pulse may be shown in Fig. 5, which is a schematic waveform diagram of the preset pulse provided by an embodiment of the present disclosure.

Example 4, as shown in Fig.5, the pulse width duty ratio of the preset pulse may be 20%, 50%, etc., and the specific pulse width duty ratio is between 10% and 99%; The pulse frequency of the preset pulse may be 0.5KHz, 1.5KHz, etc.. The specific pulse frequency is between 0.5 kHz and 3 kHz. In the above-mentioned embodiment, a modulation mode corresponding to the selection instruction is determined among a plurality of preset modulation modes, as a target modulation mode, the preset pulse may be modulated according to a modulation parameter corresponding to the target modulation mode. For example, the frequency of the modulation parameter is 28-30 Hz, the preset pulse may be modulated into a pulse with a frequency of 28-30 Hz to obtain a modulated pulse, the waveform of the modulated pulse is consistent with that corresponding to the selection instruction.

In some related schemes, only the intensity of the pulse can be adjusted, but in this embodiment, parameters such as pulse frequency and pulse intensity may be adjusted according to the user's selection instruction for pulse waveform. In this way, the user's requirements for waveform modulation can be met, and the user experience can be optimized.

In an embodiment, referring to Fig. 2, Fig. 2 is a schematic structural diagram of a control device provided by the embodiment of the present disclosure, the control device 101 may include a display screen 1011, a processor 1012 and an amplifier 1013, wherein the processor 1012 may include a processing module 1012a, a duty ratio modulation module 1012b and a filter 1012c, wherein,
the processing module 1012a, is configured to determine a gear parameter corresponding to the selection instruction among a plurality of preset gear parameters as a target gear parameter; input the target gear parameter to the duty ratio modulation module; the duty ratio modulation module 1012b, is configured to determine a duty ratio level corresponding to the target gear parameter as a target duty ratio level; output the target duty ratio level to the filter; the filter 1012c, is configured to filter the target duty ratio level to obtain a voltage value corresponding to the target gear parameter; input the voltage value to the amplifier.

Taking example 2 as an example to illustrate an alternative embodiment in the following.

### Example 2

A variety of gear parameters may be preset, such as the gear parameters of the first gear that the high level is 2V and the low level is 0.2 V; the gear parameters of the second gear that the high level is 6V and the low level is 0.6V, and so on, the specific gear parameters are not limited. A gear parameter corresponding to the selection instruction among these gear parameters is determined as the target gear parameter, for example, a gear parameter corresponding to the selection instruction that the high level is 2V and the low level is 0.2V is determined as the target gear parameter; the duty ratio level corresponding to the target gear parameter is determined, if the duty ratio level corresponding to the target gear parameter is 50% of 2V high level and 50% of 0.2V low level, then this duty ratio level may be determined as the target duty ratio level; the target duty ratio level is filtered, for example, capacitor-resistor combination filtering may be applied on the target duty ratio level to obtain the voltage value corresponding to the target gear parameter; if the duty ratio level is 50% of 2V high level, the voltage value 1.0V corresponding to the target gear parameter may be obtained by the capacitor-resistor combination filtering; if the duty ratio level is 50% of 0.2V low level, the voltage value 0.1V corresponding to the target gear parameter may be obtained by the capacitor-resistor combination filtering, etc., and the specific duty ratio level is not limited. Filtering methods for target duty ratio level may be capacitor-resistor filtering for target duty ratio level, amplitude limiting filtering for target duty ratio level, etc... The specific way of filtering the target duty ratio level is not limited; The specific voltage value corresponding to the target gear parameter is not limited. Ultimately, it is limited to human safety.

The amplifier 1013, is configured for amplifying the modulated pulse to obtain an amplified modulated pulse; sending the amplified modulated pulse to the controlled device 102.

### Example 5

The amplifier 1013 may amplify the modulated pulse according to a preset amplification factor to obtain an amplified modulated pulse, and amplifying the pulse may be understood as amplifying the intensity of the pulse. For example, the amplification factor can be preset to 2, that is, the intensity of the pulse is amplified to twice as much as the original value. If the intensity of the modulated pulse is 0.8V (volt), the intensity of the amplified modulated pulse is 1.6V.

### Example 6

After filtering the target duty ratio level to obtain the voltage value corresponding to the target gear parameter, the amplifier 1013 is specifically used to amplify the modulated pulse based on the voltage value to obtain an amplified modulated pulse; send the amplified modulated pulse to the controlled device.

The controlled device 102 is configured to vibrate or output current based on the amplified modulated pulse. Example 7 below illustrates several alternative implementations of the controlled device.

### Example 7

The controlled device 102 may be an electrode sheet, a treatment pen, etc., and the specific controlled device is not limited. In one embodiment, the controlled device is an electrode sheet, and the electrode sheet may output current based on the amplified modulated pulse. In another embodiment, the controlled device is a treatment pen, the treatment pen may determine a vibration parameter corresponding to the amplified modulated pulse among the preset vibration parameters as the target vibration parameter, after receiving the amplified modulated pulse; vibration is performed based on the target vibration parameter. The vibration parameters may be: vibration amplitude, vibration frequency, vibration duration, etc., the specific vibration parameters are not limited. The vibration amplitude is between 2µm (micron) and 500 µm, the vibration frequency is between 5Hz (Hertz) and ~500Hz, and the vibration duration is adjustable. Taking example 3 as an example to illustrate an alternative embodiment in the following.

### Example 3

If the preset vibration parameters are vibration amplitudes of 19 µm, 14µm and 12 µm; vibration frequencies of 5.4Hz, 10.7 Hz and 13.6hz; vibration durations of 15 minutes and 10 minutes; if the vibration parameters corresponding to the amplified modulated pulse are determined to be vibration amplitude of 14µm, vibration frequency of 10.7 Hz and vibration duration of 15 minutes; the treatment pen may vibrate based on vibration amplitude of 14µm, vibration frequency of 10.7 Hz and vibration duration of 15 minutes. The vibration amplitude may be 19 µm, 14 µm, 12 µm, etc., and the specific vibration amplitude is between 2 µm and 500 µm; the vibration frequency may be 5.4Hz, 10.7 Hz, 13.6 Hz, etc., the specific vibration frequency is between 5 Hz and 500 Hz; the vibration duration may be 15 minutes, 10 minutes, etc., the specific vibration duration is not limited.

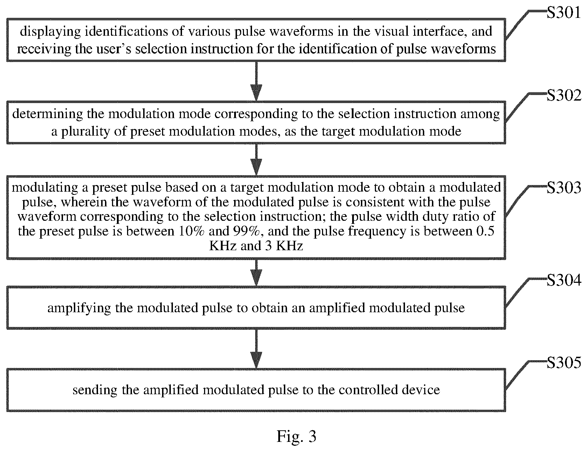

Fig. 3 is a schematic flow diagram of pulse modulation by a control device provided by an embodiment of the present disclosure, including:
S301: displaying identifications of various pulse waveforms in the visual interface, and receiving the user's selection instruction for the identifications of pulse waveforms.

The visual interface may be displayed on the display screen. The types of the display screen, the various pulse waveforms corresponding to the identifications of the various pulse waveforms displayed in the visual interface, and the selection instructions have been described above with reference to Fig. 4, and will not be repeated here.

In one embodiment, after receiving the selection instruction in S301, S302 can be directly executed.

Alternatively, in another embodiment, the selection of information such as pulse frequency and pulse intensity in the selection instruction received in S301 may be determined. After S301, the method further includes: determining a gear parameter corresponding to the selection instruction among a plurality of preset gear parameters, as a target gear parameter; determining a duty ratio level corresponding to the target gear parameter as a target duty ratio level; filtering the target duty ratio level to obtain the voltage value corresponding to the target gear parameter. The above Example 2 is also an alternative embodiment here, so it will not be repeated here.

S302: determining a modulation mode corresponding to the selection instruction among a plurality of preset modulation modes, as a target modulation mode. The above example 1 is also an alternative embodiment here, so it will not be repeated here.

S303: modulating a preset pulse based on the target modulation mode to obtain a modulated pulse, wherein the waveform of the modulated pulse is consistent with the pulse waveform corresponding to the selection instruction; the pulse width duty ratio of the preset pulse is between 10% and 99%, and the pulse frequency is between 0.5 KHz and 3 KHz.

The preset pulse is the reference pulse, and the preset pulse may be modulated according to the modulation mode corresponding to the selection instruction. The waveform of the preset pulse can refer to Fig. 5, and the above Example 4 is also an alternative implementation here, so it will not be repeated here.

S304: amplifying the modulated pulse to obtain an amplified modulated pulse.

The above examples 5 and 6 are alternative embodiments of this step, and will not be described here.

S305: sending the amplified modulated pulse to the controlled device.

The controlled devices may be electrode sheets, treatment pens, etc., and the specific controlled devices are not limited. Examples 7 and 3 introduce the implementation of alternative controlled devices, and will not be repeated here.

Corresponding to the above method embodiment, an embodiment of the present disclosure also provides a structural schematic diagram of a pulse modulation apparatus, as shown in Fig. 6, which includes:
a receiving module 701, configured for displaying the identifications of various pulse waveforms in the visual interface, and receiving the user's selection instruction for the identifications of the pulse waveforms;
a first determining module 702, configured for determining a modulation mode corresponding to the selection instruction among a plurality of preset modulation modes, as a target modulation mode;
a modulation module 703, configured for modulating a preset pulse based on the target modulation mode to obtain a modulated pulse, and the waveform of the modulated pulse is consistent with the pulse waveform corresponding to the selection instruction; the pulse width duty ratio of the preset pulse is between 10% and 99%, and the pulse frequency is between 0.5 KHz and 3 KHz;
an amplifying module 704, configured for amplifying the modulated pulse to obtain an amplified modulated pulse;
a sending module 705, configured for sending the amplified modulated pulse to the controlled device.

In an embodiment, the apparatus further comprises a second determining module, a third determining module and a filtering module (not shown in the figures), wherein,
the second determining module, configured for determining a gear parameter corresponding to the selection instruction among a plurality of preset gear parameters as a target gear parameter;
the third determination module, configured for determining a duty ratio level corresponding to the target gear parameter as a target duty ratio level;
the filtering module, configured for filtering the target duty ratio level to obtain a voltage value corresponding to the target gear parameter;

The amplifying module 704 is specifically configured for:
amplifying the modulated pulse base on the voltage value to obtain an amplified modulated pulse.

Applying the embodiments shown in the present disclosure, the preset pulse is modulated according to the modulation mode corresponding to the selection instruction of the user, and the waveform of the modulated pulse is consistent with the pulse waveform corresponding to the selection instruction; the modulated pulse is amplified and sent it to the controlled device, the controlled device vibrates or outputs current based on the amplified modulated pulse, so that the user may select a pulse waveform, the pulse waveform contains information such as pulse frequency and pulse intensity, which means that the user may select the information such as pulse frequency and pulse intensity, which satisfies the user's demand for pulse modulation and optimizes the user's experience.

The present disclosure also provides an electronic device, as shown in Fig. 7, including a processor 801 and a memory 802,
the memory 802, configured for storing computer programs;
the processor 801, configured for implementing any of the above pulse modulation methods when executing the programs stored in the memory 802.

The memory mentioned in the above-mentioned electronic device may include random access memory (RAM) or non-volatile memory (NVM), such as at least one disk memory. In some embodiments, the memory may also be at least one storage located remotely from the above processor.

The above processor may be a general-purpose processor, including a central processing unit (CPU), a network processor (NP), etc.; it may also be a digital signal processor (DSP), an application specific integrated circuit (ASIC), a field-programmable gate array (FPGA) or other programmable logic devices, discrete gate or transistor logic devices, and discrete hardware components.

In yet another embodiment provided by the present disclosure, a computer readable storage medium is also provided, in which a computer program is stored, and the computer program, when executed by a processor, realizes the steps of any of the above pulse modulation methods.

In yet another embodiment provided by the present disclosure, there is also provided a computer program product containing instructions, which, when running on a computer, causes the computer to execute the pulse modulation method of any of the above embodiments.

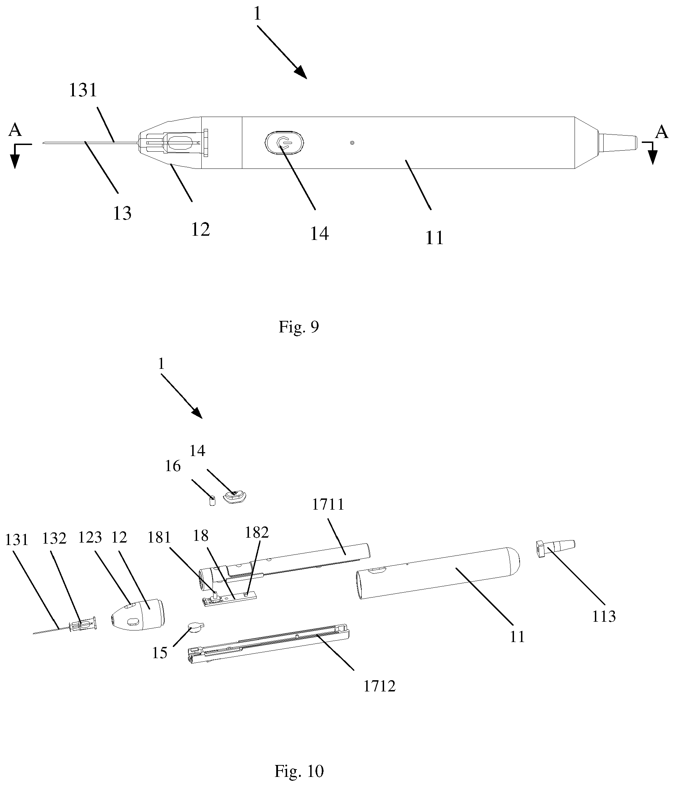

As shown in Figs. 9 and 10, the embodiment of the present disclosure provides a treatment pen 1 for a limb pain treatment instrument including a main machine 2 that outputs a vibration prescription signal to the treatment pen 1. The treatment pen 1 includes a pen rod 11 and a pen cap 12 detachably connected to a distal end of the pen rod 11. A treatment needle 13 is mounted in the pen rod 11 after the pen cap 12 is detached, and a switch button 14 is provided on the surface of the pen rod 11, a needle tube 131 of the treatment needle 13 extends out of a distal end of the pen cap 12, a vibrator 15 that can be controlled by the switch button 14 to start and stop is provided inside the pen rod 11, and the vibrator 15 is configured to, when started, transmit physical kinetic energy corresponding to a vibration prescription signal to the treatment needle 13 intervened into a lesion site.

When the limb pain treatment instrument of the embodiment of the present disclosure is used, a user (referring to a medical practitioner of a related profession) holds the treatment pen 1, and inserts the needle tube 131 of the treatment needle 13 into the lesion site of the patient, and after the treatment needle 13 generates physical kinetic energy, the treatment of the lesion site may be achieved. In the embodiment of the present disclosure, the physical kinetic energy may refer to the kinetic energy generated by physical vibration. The "proximal end" may be understood as the end of the treatment pen or some part of the treatment pen closer to the medical practitioner of the related profession, and the "distal end" may be understood as the end of the treatment pen or some part of the treatment pen that is further away from the medical practitioner of the related profession.

The pen rod 11 and the pen cap 12 of the treatment pen 1 are detachably connected. When the treatment pen 1 is idle, the treatment needle 13 is usually not installed to avoid stabbing the medical practitioner of the related profession or polluting the treatment needle 13. When the lesion needs to be treated, the pen cap 12 may be detached from the pen rod 11, the treatment needle 13 may be mounted on the mounting bracket 17 of the pen rod 11, and then the pen cap 12 may be fixed to the pen rod 11. At this time, the needle tube 131 extends out of the needle tube exit hole 122 formed at the distal end of the pen cap 12, and extends out of the pen cap 12. The treatment needle 13 may be a specialized treatment needle or a general disposable syringe needle, and used to intervene into the lesion site formed by soft tissue damage for diagnosis and/or treatment. The treatment needle has the advantage of tough texture, high accuracy, simple installation, convenient replacement. After the treatment needle 13 intervenes into the lesion, the skin wound is small, causing little pain to the patient, and the recovery is rapid.

A switch button 14 is provided on the surface of the pen rod 11, which may be a push-type or sliding-type structure. The switch button 14 may control the start and stop of the vibrator 15. Therefore, the switch button 14 may be directly or indirectly electrically connected to the vibrator 15, and when the vibrator 15 is started, physical kinetic energy may be generated. The vibrator 15 is directly or indirectly in contact with the treatment needle 13, or the vibrator 15 is directly or indirectly fixedly connected to the treatment needle 13, so that the vibrator 15 may transfer physical kinetic energy to the treatment needle 13. When the treatment needle 13 intervenes into the lesion site, it may bring physical kinetic energy into the lesion site, so that the lesions such as adhesion nodules may be better loosened, thus realizing the treatment of the lesion site, and achieving the purpose of improving the pain or even eliminating the lesion.

The embodiment of the present disclosure provides a treatment pen 1 for a limb pain treatment instrument, the limb pain treatment instrument includes a treatment pen 1 and a main machine 2, a touch display screen 23 is arranged on the surface of a chassis 21 of the main machine 2, so that a user may select a vibration prescription via the touch display screen 23. The main control board of CPU 24 may output a vibration prescription signal corresponding to the vibration prescription to the vibrator 15 of the treatment pen 1. The vibrator 15 can generate physical kinetic energy corresponding to the vibration prescription signal when it is started and receives the vibration prescription signal, and transmit the physical kinetic energy to the treatment needle 13 installed at the front end of the pen rod 11. When the treatment needle 13 intervenes into the lesion site for treatment, the physical kinetic energy is intervened into the lesion site with the treatment needle, so that the lesions such as damaged adhesion nodules may be better loosened to realize the treatment of the lesion site, and thus achieve the purpose of improving pain or even eliminating the lesion.

As shown in Fig. 10, the distal end of the pen rod 11 is provided with an elastic wave bead 16 for mounting and releasing the pen cap 12, wherein the number of elastic wave bead 16 may be at least one. Specifically, the outer surface of the elastic wave bead 16 is provided with an external thread, and the distal end of the pen rod 11 is provided with a threaded hole. The elastic wave bead 16 is fixed in the threaded hole of the pen rod 11 through the external thread. Since the wave bead of the elastic wave bead 16 can compress the spring when it is under pressure, when the pen cap 12 is mounted on the pen rod 11 through the elastic wave bead 16, the spring within the elastic wave bead 16 is in a compressed state, that is, provides a pressure for the pen cap 12 such that a friction force is generated between the pen cap 12 and the elastic wave bead 16, thereby fixedly connecting the pen cap 12 and the pen rod 11. When the pen cap 12 needs to be detached, the elastic wave bead 16 may release the pen cap 12 by applying a force greater than the friction force on the pen cap 12, so that the pen cap 12 may be detached from the pen rod 11. The above technical solution is simple in structure and convenient to operate.

A contact surface 121 is provided within the pen cap 12, and the contact surface 121 is located at the distal end of the pen cap 12. The treatment needle 13 further includes a needle tube holder 132 fixedly connected to the needle tube 131. When the treatment needle 13 is fixed on the pen rod 11, the distal end surface of the needle tube holder 132 contacts the contact surface 121. In this way, the contact surface 121 inside the pen cap 12 can limit the movement of the treatment needle 13 along the length direction of the pen rod 11, thus improving the mounting stability of the treatment needle 13 and reducing the movement of the treatment needle 13 in the process of physical kinetic energy, which in turn can improve the safety of the treatment needle 13 when intervening into the lesion site for treatment.

In addition, in order to further improve the safety of the use of the treatment needle 13, the distal end of the pen cap 12 is provided with a needle tube exit hole 122. After the treatment needle 13 and the pen cap 12 are fixed to the pen rod 11, the needle tube 131 passes through the needle tube exit hole 122 and extends out of the distal end of the pen cap 12, and the needle tube exit hole 122 and the needle tube 131 are gap fit, so that the needle tube exit hole 122 may limit the lateral displacement of the treatment needle 13, thereby further improving the safety of the use of the treatment needle 13.

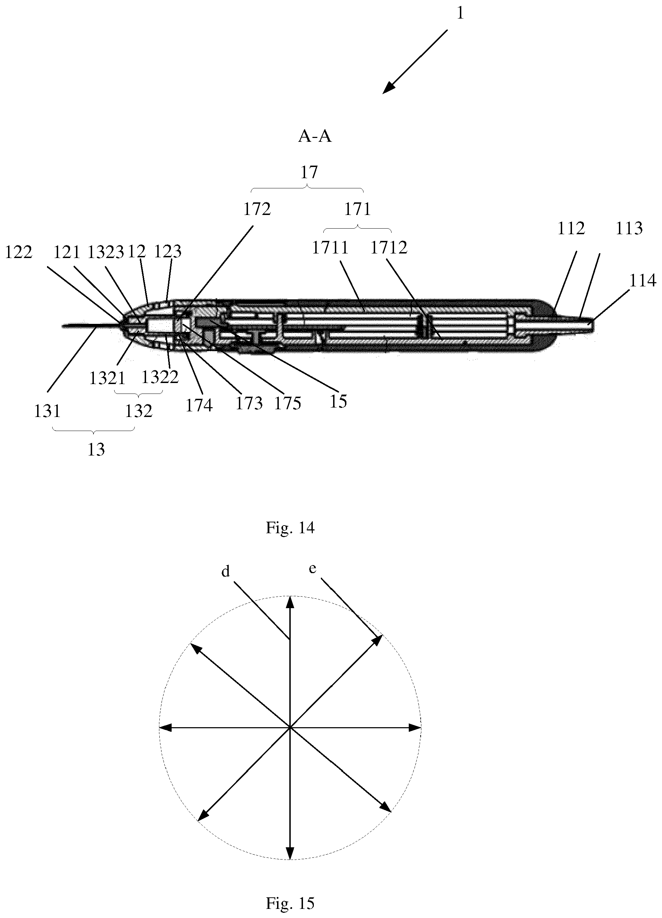

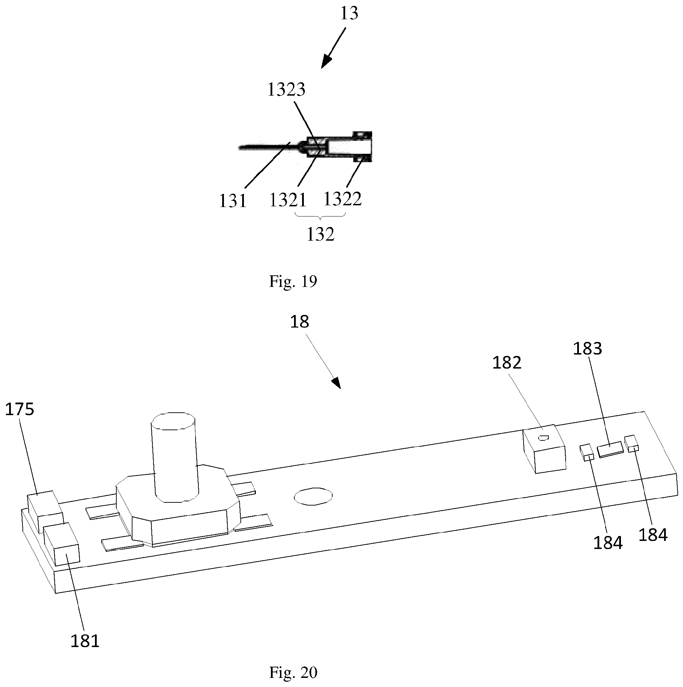

In some embodiments, as shown in Figs. 19 and 14, the needle tube holder 132 includes a needle tube fixing part 1321 and a clamping part 1322 fixedly connected to the needle tube fixing part 1321. The needle tube fixing part 1321 is provided with a needle tube mounting hole 1323 penetrating through the needle tube fixing part, the needle tube 131 is inserted and fixed within the needle tube mounting hole 1323. The clamping part 1322 is annular and hollow, and a cavity of the clamping part 1322 communicates with the needle tube mounting hole 1323. Since the needle tube 131 is inserted and fixed in the needle tube mounting hole 1323, the cavity of the clamping part 1322 communicates with the needle tube mounting hole 1323, so that the cavity of the clamping part 1322 also communicates with the needle tube 131. Here, the material of the needle tube 131 may be medical stainless steel, and the material of the needle tube fixing part 1321 may be medical plastic, specifically may be transparent medical plastic.

As shown in Fig. 10, the treatment pen 1 further includes a mounting bracket 17 arranged inside the pen rod 11, the mounting bracket 17 includes a bracket body 171 extending along the length direction of the pen rod 11 and a mastoid 172 fixedly connected to the distal end of the bracket body 171, and the clamping part 1322 is sleeved and fixed on the mastoid 172. The mounting bracket 17 and the pen rod 11 may be of one-piece molded structure, or the mounting bracket 17 and the pen rod 11 may be fixedly connected.

The cross section of the pen rod 11 is annular, so the pen rod 11 has a cavity inside, and the mounting bracket 17 may be arranged within the cavity of the pen rod 11. The mounting bracket 17 includes a bracket body 171 extending along the length direction of the pen rod 11 and a mastoid 172 fixedly connected to the distal end of the bracket body 171, wherein the mounting bracket 17 may be wholly located in the cavity inside the pen rod 11, that is, both the bracket body 171 and the mastoid 172 are located in the cavity inside the pen rod 11, and the distal end of the mastoid 172 does not exceed out of the distal end of the pen rod 11; the mounting bracket 17 may be partially located in the cavity within the pen rod 11, that is, the bracket body 171 is located in the cavity within the pen rod 11, and the distal end of the bracket body 171 and the distal end of the pen rod 11 are located in a same plane, and the mastoid 172 extends out of the distal end of the pen rod 11.

Since the clamping part 1322 of the needle tube holder 132 is annular and hollow, the cross section of the outer surface of the mastoid 172 fixedly connected to the clamping part 1322 may also be circular, and the clamping part 1322 and the mastoid 172 may be in an interference fit or a transition fit. In this way, the treatment needle 13 may be more stably fixed to the pen rod 11, thus further improving the safety of the use of the treatment pen 1.

In some embodiments, as shown in Fig. 14, the bracket body 171 includes a first body part 1711 and a second body part 1712 which are oppositely arranged and fixedly connected, and the vibrator 15 is fixedly connected between the first body part 1711 and the second body part 1712. Both the cross-sections of the first body part 1711 and the second body part 1712 may be semi-annular, and the bracket body 171 formed by fixedly connecting these two parts is annular and hollow, so that it may be better fixedly connected to the pen rod 11. In addition, the first body part 1711 may be clamped to the second body part 1712. When the treatment pen 1 is assembled, the vibrator 15 may be fixedly connected to the first body part 1711, and then the first body part 1711 and the second body part 1712 may be fixedly connected. Therefore, this structure not only makes the internal structure of the treatment pen more compact, but also facilitates the assembly.

Since there is a hollow structure between the first body part 1711 and the second body part 1712, bottom films may be provided at the distal ends of the first body part 1711 and the second body part 1712, and the mastoid 172 is fixedly connected to the first body part 1711 and the second body part 1712 through the bottom films, which is made of transparent material.

In addition, a vibrator 15 may be fixedly connected between the first body part 1711 and the second body part 1712. After the vibrator 15 generates physical kinetic energy, the physical kinetic energy may be transferred to the first body part 1711 and the second body part 1712, and then the physical kinetic energy is transferred to the mastoid 172 and the treatment needle 13.

The mounting bracket 17 further includes a side wall 173 fixed to the distal ends of the first body part 1711 and the second body part 1712 and surrounding the mastoid 172, and the side wall 173 is in contact with the outer side surface of the needle tube holder 132. That is, an annular groove 174 is formed between the mastoid 172 and the side wall 173. After the needle holder 132 is fixed on the mastoid 172, the needle holder 132 is also located in the annular groove 174, and the side wall 173 is in contact with the outer side surface of the needle holder 132. Thus, the mastoid 172 and the side wall 173 may better fix and limit the displacement of the needle holder 132. Therefore, the treatment needle 13 can be more stably fixed on the needle tube 131, thereby further improving the use safety of the treatment pen 1.

In some embodiments, as shown in Fig. 14, an ultraviolet disinfection band LED emitter 175 which emits light toward the treatment needle 13 is arranged at the distal end inside the pen rod 11, and is configured for ultraviolet disinfection of the treatment needle 13, the mastoid 172, the side wall 173 and the pen cap 12. The ultraviolet disinfection band LED emitter 175 may be fixed to the bracket body 171 or integrated on the PCB printed control board 18, and the light emitting direction is in the direction of the treatment needle 13. Since a mastoid 172 for fixing the treatment needle 13 is provided on the bracket body 171, the mastoid 172 may be provided with a light transmission hole penetrating through the mastoid. In this way, the ultraviolet light emitted by the ultraviolet disinfection band LED emitter may irradiate to the treatment needle 13, the mastoid 172, the side wall 173 and the pen cap 12, thereby performing ultraviolet disinfection on the treatment needle 13, the mastoid 172, the side wall 173 and the pen cap 12, and further keeping the treatment needle 13, the mastoid 172, the side wall 173 and the pen cap 12 clean. Since the bottom films made of transparent materials are provided at the distal ends of the first body part 1711 and the second body part 1712, the bottom films may prevent the effusion from flowing to the ultraviolet disinfection band LED emitter.

In some embodiments, as shown in Fig. 10, the pen cap 12 is provided with at least one sight window 123 along the circumferential direction, and the needle tube holder 132 is a needle tube holder 132 made of transparent material, and the needle tube holder 132 can be observed through each sight window 123. In order to achieve the purpose of being able to observe the needle tube holder 132 through each sight window 123, the sight window 123 is arranged on the pen cap 12 at a position corresponding to the needle tube holder 132. In the process of using the treatment pen 1 to treat the lesion site, after the treatment needle 13 is inserted into the lesion site, if there is effusion in the lesion site, the effusion will flow through the needle tube 131 to the needle tube holder 132 under the action of the internal pressure of the lesion site, and the medical practitioner of related profession may observe whether there is effusion in the patient's lesion site through the sight window 123, which is convenient to determine further treatment plans. Moreover, the sight window 123 arranged on the pen cap 12 may provide safety protection, facilitate observation, stable installation, safety and health, smooth installation and convenient treatment. The material of the pen cap 12 comprises alloy material.

In some embodiments, as shown in Fig. 10, a PCB (Printed Circuit Board) control board 18 is arranged inside the pen rod 11, and an LED backlight 181, a vibration indicator lamp 182 and a switch button 14 are integrated on the PCB control board 18. The LED backlight 181 and the switch button 14 are exposed on the surface of the pen rod 11, and a working observation hole is also arranged on the surface of the pen rod 11. The working observation hole corresponds to the position of the vibration indicator lamp 182, and the LED backlight 181 is closer to the distal end of the pen rod 11 than the vibration indicator lamp 182. The PCB control board 18 is also electrically connected to the vibrator 15, specifically, the vibrator 15 and the PCB control board 18 are fixed by soldering.

The LED backlight 181 is integrated into the PCB control board 18, and the LED backlight 181 is closer to the distal end of the pen rod 11 than the vibration indicator lamp 182. In this way, the LED backlight 181 may provide lighting for the medical practitioner of related profession during the treatment of the lesion site with the treatment pen 1, thus enabling the medical practitioner of related profession to observe more clearly whether there is effusion in the needle tube holder 132 through the sight window 123.

In addition, the LED backlight 181 is exposed on the surface of the pen rod 11, which may be realized by setting the pen rod 11 and the bracket body 171 as transparent materials, or by setting mounting holes in the bracket body 171 and the pen rod 11 so that the LED backlight 181 protrudes from the surface of the pen rod 11.

The switch button 14 may be a push-type structure or a toggle-type structure. Since a medical practitioner of a related profession is required to press or toggle the switch button 14, the switch button 14 needs to protrude from the surface of the pen rod 11.

In some embodiments, as shown in Fig. 14, the proximal end of the pen rod 11 is provided with a cable hole 112, and a cable clamp 113 is sleeved on the cable hole 112. The cable connected to the PCB control board 18 passes through the cable hole 112 and is fixed by the cable clamp 113, and is electrically connected to the treatment pen output interface 22 of the main machine 2, that is, one end of the cable is electrically connected to the main machine 2, and the other end of the cable passes through the cable hole 112 and is electrically connected to the PCB control board 18, the PCB control board 18 is used to receive the vibration prescription signal from the CPU main control board 24 and output it to the vibrator 15. The cable card 113 may also be provided with a cable fixing hole 114 penetrating through the cable card, and the cable connected to the PCB control board 18 may pass through the cable fixing hole 114 of the cable card 113 and be connected to the treatment pen output interface 22 of the main machine 2. The material of the cable card 113 may usually be plastic or rubber, which may protect the cable and reduce the cable damage caused by the friction between the cable and the cable hole 112 of the treatment pen 1. The main control board 24 may output the vibration prescription signal to the PCB control board 18 through the cable, and then the PCB control board 18 controls the vibration frequency, amplitude and vibration speed of the physical kinetic energy of the vibrator 15 according to the vibration prescription signal.

In other embodiments, the proximal end of the pen rod 11 is provided with a treatment pen input interface, and the treatment pen input interface is electrically connected to the treatment pen output interface 22 of the main machine 2 through a detachable cable; the PCB control board 18 is also electrically connected to the vibrator 15 and the treatment pen input interface, respectively. The PCB control board 18 is used to receive the vibration prescription signal from the CPU main control board 24 and output it to the vibrator 15. When the treatment pen 1 is needed to treat the lesion site, one cable may be used to connect the treatment pen input interface of the treatment pen 1 to the treatment pen output interface 22 of the main machine 2. When the main machine 2 is not needed, the cable can be pulled out and stored.

In some embodiments, a trace of vibration output by the vibrator 15 is in a plane perpendicular or parallel to an axis of the pen rod 11, and the trace of the vibration includes a trace of reciprocating motion in a single direction, and/or a trace of reciprocating motion in multiple angular directions cyclically centred on the axis of the pen rod 11. Both the single direction and the plurality of angular directions are in a plane perpendicular or parallel to the axis of the pen rod 11, and the single direction may be any direction in a plane perpendicular or parallel to the axis of the pen rod 11. By changing the installation direction of the vibrator 15 in the pen rod 11, the vibrator 15 may obtain vibration traces with different single directions.

The trace of reciprocating motion in multiple angular directions cyclically, specifically can be, circularly reciprocate in multiple angular directions clockwise or counterclockwise. Fig. 15 is a schematic diagram of the vibration trace of a vibrator in some embodiments of the present disclosure. As shown in Fig. 15, for multiple angular directions, it is possible to perform linear reciprocating motion along a first angular direction d first, then perform linear reciprocating motion along a second angular direction e according to clockwise, and then perform linear reciprocating motion along the first angular direction d again after the linear reciprocating motion in multiple angular directions is completed, and so on.

In addition, the center of gravity of the vibrator 15 and the center of gravity of the treatment pen 1 may coincide with the axis of the pen rod 11, which can reduce the situation where centrifugal vibration generates a bias load that causes excessive amplitude of the vibrator 15 resulting in the damage of the lesion site.

In some embodiments, the vibrator 15 is a mechanical vibrator 15, the amplitude of the vibrator 15 is designed between 2 µm and 500 µm, and the vibration frequency of the vibrator 15 is designed between 5 Hz and 500 Hz. The amplitude of the vibrator 15 is limited within the aforementioned range, which can avoid additional damage to the lesion site due to excessive vibration amplitude in the lesion site.

In addition, the physical kinetic energy generated by the vibration prescription is brought into the lesion site by the treatment needle 13, so that the blood and body fluid circulation and metabolic functions of the lesion site are improved; moreover, since the diameter of red blood cells is about seven micrometres (microns), and the amplitude of physical kinetic energy brought in by the treatment needle 13 is larger than the diameter of red blood cells, in the process of treatment, the metabolism and exchange of harmful substances among tissues with oxygen metabolism of red blood cells smoothly, and tiny particles of harmful substances can be metabolized from the painful focus, thus accelerating the healing of the lesion site, thus achieving the purpose of treatment.

In order to facilitate medical staff to hold the treatment pen 1, the outer diameter of the pen rod 11 of the treatment pen 1 is between 5mm and 50mm, the distance between the distal end of the pen cap 12 and the proximal end of the pen rod 11 is between 30mm and 300mm, the distance between the distal end of the pen cap 12 and the vibration signal input interface is between 30mm and 350mm, and the distance between the distal end of the treatment needle 13 and the proximal end of the pen rod 11 is between 45mm and 400mm, the distance between the center of the switch button 14 and the distal end of the pen cap 12 is between 10 mm and 100 mm. In this way, it is more convenient for medical staff to hold and operate, and the operation error caused by excessive finger action when pressing the switch button 14 is reduced.

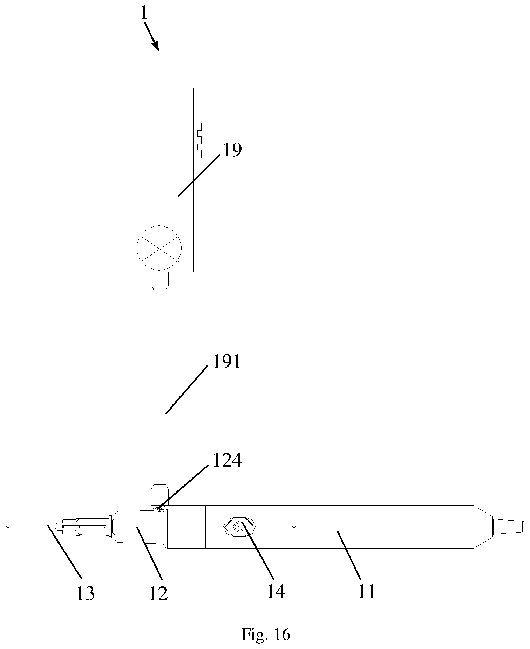

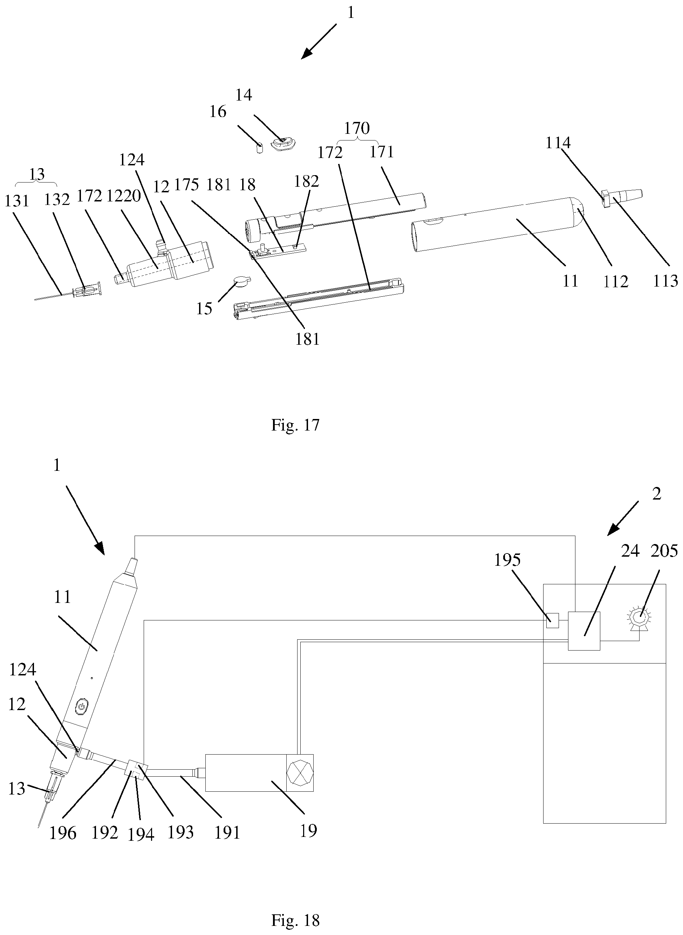

In some embodiments, as shown in Figs. 16 and 17, the treatment pen 1 further comprises a negative pressure pump 19 detachably connected to the distal end of the pen rod 11, and the pen cap 12 is made of transparent material. A joint 124 is fixed on the surface of the pen cap 12, and the negative pressure pump 19 is connected to the joint 124 through a drainage tube 191, and the joint 124 is communicated with the pen cap 12 and the treatment needle 13. The negative pressure pump 19 is used for providing suction negative pressure for the effusion within the lesion site when being started, so that the effusion can be pumped out from the lesion site. The joint 124 may be detachably connected to the pen cap 12, welded to the pen cap 12, and molded with the pen cap 12 at one time.

The negative pressure pump 19 is provided with a physical entity switch, which can be operated by medical practitioner of related profession to control the start and stop of the negative pressure pump 19. In addition, the negative pressure pump 19 may have a built-in power supply, or it may be electrically connected to the main machine, the negative pressure pump 19 is supplied power through the main machine and the start and stop of the negative pressure pump 19 is controlled by the main machine.

The negative pressure pump 19 is communicated with the joint 124 through the drainage tube 191, and the drainage tube 191 may not be installed on the joint 124 before treating the lesion site. In this case, a detachable rubber plug may be provided on the surface of the joint 124, and the joint 124 may be exposed after the rubber plug is detached. When the joint 124 is not in use, the rubber plug may be better waterproof and dustproof.

When the treatment needle 13 is intervened into the lesion site of the patient, if there is effusion in the lesion site, the effusion will flow into the cavity inside the pen cap 12 through the treatment needle 13 under the action of negative pressure inside the lesion site. Since the pen cap 12 is made of transparent material, medical practitioner of related profession may determine whether there is effusion in the lesion site of the patient by observing whether there is effusion in the pen cap 12.

When there is no effusion accumulation in the pen cap 12, the switch button 14 may be directly operated, and the vibrator 15 may be started to generate physical kinetic energy according to the vibration prescription signal, and the physical kinetic energy may be transmitted to the treatment needle 13, so that the treatment needle 13 may bring the physical kinetic energy into the lesion site for treatment. When the treatment for the lesion site is finished, the switch button 14 may be stopped, the vibrator 15 stops vibrating, then the treatment needle 13 is pulled out from the lesion site, the vibration prescription is selected on the touch display screen 23 again, and the above operations are continued until treatment for all the lesion sites of the patient is finished.

When it is observed that there is effusion in the pen cap 12, it indicates that there is effusion in the lesion site, so the negative pressure pump 19 needs to be used to pump the effusion. At this time, the physical entity switch on the negative pressure pump 19 may be turned on first, the negative pressure pump 19 provides negative pressure for the effusion at the lesion site. Under the negative pressure, the effusion flows through the treatment needle 13, the pen cap 12, the joint 124, the drainage tube 191 and the negative pressure pump 19 in sequence from the lesion site, and finally to the containing bottle placed at the outlet of the negative pressure pump 19. Then, the switch button 14 is operated, the vibrator 16 is started, and the physical kinetic energy is transmitted to the treatment needle 13, so that the treatment needle 13 brings the physical kinetic energy into the lesion site for treatment. Alternatively, the switch button 14 may be operated first, the vibrator 16 is started, and the physical kinetic energy is transmitted to the treatment needle 13, so that the treatment needle 13 brings the physical kinetic energy into the lesion site for treatment, after the vibration treatment of the lesion site is completed; then, the physical entity switch of the negative pressure pump 19 is turned on, and the negative pressure pump 19 is used to perform suction treatment on the effusion at the lesion site.

In the process of suction of the effusion by the negative pressure pump 19, when it is found that there is no more effusion in the pen cap 12, it indicates that the effusion in the lesion site has been sucked up. At this time, the physical entity switch of the negative pressure pump 19 may be operated, and the negative pressure pump 19 may be turned off to complete the process of suction of the effusion.

The treatment pen 1 for the limb pain treatment instrument provided by the embodiments of the present disclosure includes the pen rod 11, the pen cap 12 detachably connected to the distal end of the pen rod 11, and the negative pressure pump 19. The vibrator 15 arranged inside the pen rod 11 can generate physical kinetic energy corresponding to the vibration prescription signal when it is started and receives the vibration prescription signal, and transmit the physical kinetic energy to the treatment needle installed at the front end of the pen cap 12. When the treatment needle 13 intervenes into the lesion site for treatment, the physical kinetic energy is intervened into the lesion site with the treatment needle 13, so that the lesions such as damaged adhesions and nodules may be better loosened to realize the treatment of the lesion site, and thus achieve the purpose of improving pain or even eliminating the lesion. In addition, the negative pressure pump 19 may provide suction negative pressure for the effusion in the lesion site when it is started, so that the effusion may be drawn out from the lesion site under the action of this negative pressure. Therefore, the treatment pen 1 may also perform suction treatment on the effusion in the lesion site, which may provide better treatment effect on the lesion site.

In addition, the treatment pen 1 for the limb pain treatment instrument provided by some embodiments of the present disclosure is also able to treat the resulting associated limb pain and discomfort such as bone lesions and joint pain, such as cervical spondylosis, scapulohumeral periarthritis, lumbar spondylosis or arthritis, etc..

In some embodiments, as shown in Fig. 18, the treatment pen 1 further includes a communication tube 192 that communicates the drainage tube 191 and the joint 124; a control signal for starting and stopping the negative pressure pump 19 comes from the main machine 2, and the main machine 2 generates a corresponding control signal by detecting whether there is effusion at a position where a sensor 193 in the communication tube 192 is located.

The inner and outer surfaces of the communication tube 192 are cylindrical, and the communication tube 192 may directly or indirectly communicate with the joint 124. When the communication tube 192 directly communicates with the joint 124, one end of the communication tube 192 is connected to the joint 124, and the other end is connected to the drainage pipe 191. The outer surface of the joint 124 is usually provided with a screw thread or other type of connection surface, or the inner surface of the communication tube 192 may also be provided with a connection surface matching with the connection surface of the joint 124, and the joint 124 is fixedly connected with the communication pipe 192 by the mating connection between the connection surface of the joint 124 and the inner surface of the communication tube 192. The other end of the communication tube 192 is provided with a boss, and the drainage tube 191 is sleeved on the outer surface of the boss to communicate the drainage tube 191 with the communication tube 192. When the communication tube 192 is indirectly communicated with the joint 124, the communication tube 192 and the joint 124 may be communicated through a hose 196.

A sensor 193, which may be a pressure sensor or a liquid level sensor, is arranged inside the communication tube 192. The sensor 193 is electrically connected to the CPU main control board 24. The sensor 193 may detect whether there is effusion at the position of the sensor 193 in the communication tube 192, and generate a corresponding electrical signal, and transmit the electrical signal to the CPU main control board 24. The CPU main control board 24 may receive the electrical signal sent by the sensor 193, the electrical signal may be an electrical signal corresponding to the presence of effusion at the position of the sensor 193 in the communication tube 192, or an electrical signal corresponding to the absence of effusion at the position of the sensor 193 in the communication tube 192. The CPU main control board 24 may determine whether there is effusion at the position of the sensor 193 in the communication tube 192 according to the electrical signal, and if there is effusion, it indicates that the effusion in the lesion has not been completely sucked up. If there is no effusion, it indicates that the effusion in the lesion site has been sucked up, and the negative pressure pump 19 is no longer required to suction the effusion in the lesion site. Since the CPU main control board 24 is also electrically connected to the negative pressure pump 19, the CPU main control board may send a control signal to the negative pressure pump 19 to control the negative pressure pump 19 stop. Then the negative pressure pump 19 stops the suction process of the effusion. This technical solution increases the safety of the use of the treatment pen 1.

In addition, a buzzer 205 or an alarm lamp may be installed in the main machine 2. When the electric signal received by the CPU main control board 24 indicates that there is no effusion at the position of the sensor 193 in the communication tube 192, in addition to sending a control signal to the negative pressure pump 19, it may also send a control signal to the buzzer 205 or the alarm lamp to make the buzzer 205 beep or make the alarm lamp light up, so as to remind the medical practitioner of related profession that the effusion has been sucked up.

In some embodiments, as shown in Fig. 18, the sensor 193 is a liquid level sensor 193, which includes two electrodes 194 fixed to an inner wall of the communication tube 192 with a gap, and a control circuit board 195 electrically connected to the two electrodes 194, respectively. The control circuit board 195 is arranged in the main machine 2, and the control circuit board 195 is also electrically connected to the CPU main control board 24. Both electrodes 194 may be arranged at one end of the communication tube 192 close to the joint 124 and on the same plane perpendicular to the axis of the communication tube 192.

When there is effusion at the position where the two electrodes 194 are located in the communication tube 192, the two electrodes 194 are conducted, and the control circuit board 195 transmits an electrical signal corresponding to the conduction of the two electrodes 194 to the CPU main control board 24; or, when there is no effusion at the position where the two electrodes 194 are located in the communication tube 192, the two electrodes 194 are disconnected, and the control circuit board 195 transmits an electrical signal corresponding to the disconnection of the two electrodes 194 to the CPU main control board 24.

The electrode 194 in the liquid level sensor 193 may be an electrode sheet or an electrode needle. Both electrodes 194 are connected to the control circuit board 195. Since the effusion in the lesion site has a conductive function, when there is effusion within the communication tube 192 at the position where the electrodes 194 are located, the two electrodes 194 are conducted, and the two electrodes 194, the control circuit board 195, and the cable connecting the electrodes 194 and the control circuit board 195 form a closed loop, there is current flows through the control circuit board 195, the CPU main control board 24 electrically connected to the control circuit board 195 may receive electrical signals. When there is no effusion at the position where the electrode 194 are located in the communication tube 192, the two electrodes 194 are disconnected, and the control circuit board 195 transmits an electrical signal corresponding to the disconnection of the two electrodes 194 to the CPU main control board 24. When the signal received by the CPU main control board 24 indicates that there is no effusion within the communication tube 192 at the position where the electrode 194 is located, it indicates that the effusion in the lesion site has been sucked up, and a control signal for controlling the stop of the negative pressure pump 19 may be sent to the negative pressure pump 19, and the negative pressure pump 19 stops providing negative pressure. With the above structure, the automation degree of the treatment pen may be improved, and the use safety may be further improved, and the situation that the negative pressure pump 19 continues to provide negative pressure to the lesion site after the effusion has been sucked up due to the negligence of the medical practitioner of related profession can be reduced.

As shown in Fig. 17, in other embodiments, the distal end of the pen cap 12 is provided with a mastoid 172, and the clamping part 1322 is sleeved and fixed on the mastoid 172. A through hole 1220 is provided on the mastoid 172, and the clamping part 1322 communicates with the pen cap 12 through the through hole 1220.

Since the clamping part 1322 of the needle tube holder 132 is annular and hollow, the cross section of the outer surface of the mastoid 172 fixedly connected to the clamping part 1322 may also be circular, and the clamping part 1322 and the mastoid 172 may be in an interference fit or a transition fit. In this way, the treatment needle 13 may be more stably fixed to the pen cap 12, thus further improving the safety of the use of the treatment pen 1.

The through hole 1220 is provided on the mastoid 172, so that the clamping part 1322 may communicate with the inner cavity of the pen cap 12 through the through hole 1220, thus making the needle tube 131 communicated with the needle tube base 132, the inner cavity of the pen cap 12 and the joint 124.

In other embodiments, as shown in Fig. 17, the treatment pen 1 further comprises a bracket 170 arranged inside the pen rod 11, and the bracket 170 comprises a first body part 1711 and a second body part 1712 which are oppositely arranged and fixedly connected, and the vibrator 15 is fixedly connected between the first body part 1711 and the second body part 1712. The bracket 170 and the pen rod 11 may be of one-piece molded structure, or the bracket 170 and the pen rod 11 may be fixedly connected. The cross section of the pen rod 11 is annular, so the pen rod 11 has a cavity inside, and the bracket 170 may be arranged within the cavity of the pen rod 11.

In addition, the first body part 1711 may be clamped to the second body part 1712, and the vibrator 15 may be fixedly connected between the first body part 1711 and the second body part 1712. After the vibrator 15 generates physical kinetic energy, the physical kinetic energy may be transmitted to the first body part 1711 and the second body part 1712, and then to the pen rod 11 and the treatment needle 13. When assembling the treatment pen 1, the vibrator 15 may be fixedly connected to the first body part 1711, and then the first body part 1711 and the second body part 1712 may be fixedly connected. Therefore, this structure not only makes the internal structure of the treatment pen more compact, but also facilitates assembly.

In some embodiments, the vibrator 15 includes a rotary centrifugal vibrator 150. The rotary centrifugal vibrator 150 is used to transmit the physical kinetic energy corresponding to the vibration prescription signal to the treatment needle 13 when it is started.

As shown in Fig. 9, Fig. 10 and Fig. 20, in some embodiments, when the rotary centrifugal vibrator 150 which can be controlled by the switch button 14 to start and stop is arranged inside the pen rod 11, the treatment pen 1 further comprises a PCB control board which is electrically connected to the rotary centrifugal vibrator 150 and used for outputting a vibration prescription signal to the rotary centrifugal vibrator 150, and a common mode filter is integrated on the PCB control board.

The way in which the PCB control board 18 is electrically connected to the rotary centrifugal vibrator 150 is not limited, specifically, the vibrator 15 may be welded and fixed with the PCB control board 18. The above common mode filter includes a common mode inductor 183 and a filter capacitor 184, and the above physical kinetic energy may be kinetic energy generated by physical vibration.

According to the treatment pen provided by the embodiments of the present disclosure, the rotary centrifugal vibrator 150 can generate physical kinetic energy corresponding to the vibration prescription signal when it is started and receives the vibration prescription signal, and transmit the physical kinetic energy to the treatment needle 13 installed at the front end of the pen rod 11. When the treatment needle 13 intervenes into the lesion site for treatment, the physical kinetic energy is intervened into the lesion site with the needle, so that the lesions such as the damaged adhesion nodules may be better loosened to realize the treatment of the lesion site, and thus achieve the purpose of improving pain or even eliminating the lesion. The trace of the output vibration of the rotary centrifugal vibrator 150 lies in the plane perpendicular or parallel to the axis of the pen rod 11, the vibration trace of the treatment needle includes a trace of reciprocating motion in multiple angular directions with the axis of the penholder 11 as the center. Compared with reciprocating motion in single direction, it has sufficient vibration and better therapeutic effect. In addition, the common mode inductor 183 and the filter capacitor 184 can suppress or eliminate the electromagnetic interference clutter generated by the rotary centrifugal vibrator 150 during operation, so as to avoid affecting the normal operation of the treatment pen.

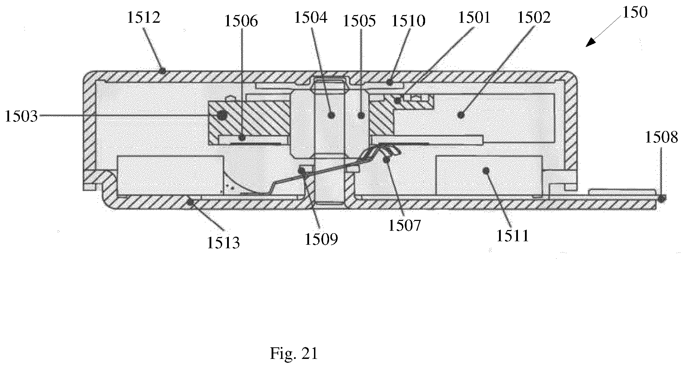

In the treatment pen 1 provided by the present disclosure, the rotary centrifugal vibrator 150 is not particularly limited, and may be a conventional rotary centrifugal vibrator in the field. As shown in Fig. 21, specifically, The rotary centrifugal vibrator 150 includes a coil 1501 for generating a magnetic field after being energized, an osicllator 1502 playing an eccentric role during rotation, a rotor 1503, a shaft 1504, a bearing 1505 for supporting the rotor to rotate and generate vibration friction, a hard plate 1506 containing a commutator, a brush 1507 for energizing and supporting the rotor, a soft plate 1508 for supporting the brush and conducting current, a washer 1509 for controlling the height of the rotor, a viscous gasket 1510 to reduce the friction between the bearing and the upper casing, a magnetic steel 1511 to generate a permanent magnetic field after magnetization, an upper casing 1512 and a lower casing 1513. The osicllator 1502 installed on the rotor 1503 makes the center of gravity of the rotor 1503 deviate from the center of gravity of the shaft, and the center of gravity constantly changes in the process of rotation, resulting in vibration.

In the treatment pen 1 provided by the present disclosure, the common mode inductor 183 and the filter capacitor 184 may be selected according to the actual requirements, as long as the electromagnetic interference clutter generated by the filter rotary centrifugal vibrator 150 during operation can be effectively suppressed or eliminated, so that the treatment pen 1 may work stably and effectively.

In some embodiments of the present disclosure, at least one of the treatment needle 13, the pen cap 12 and the pen rod 11 has terahertz energy, and the frequency of the terahertz energy is 0.1 THz -10 THz.

According to the treatment pen 1 provided by the embodiment of the present disclosure, the vibrator 15 can generate physical kinetic energy corresponding to the vibratory prescription signal when it is started and receives the vibration prescription signal, and transmit the physical kinetic energy to the treatment needle 13 installed at the front end of the pen rod 11. When the treatment needle 13 intervenes into the lesion site for treatment, the physical kinetic energy is intervened into the lesion site with the needle, so that the lesions such as the damaged adhesions and nodules may be better loosened to realize the treatment of the lesion site, and thus achieving the purpose of improving pain or even eliminating the lesion. At the same time, since at least one of the treatment needle 13, the pen cap 12 or the pen rod 11 of the treatment pen 1 has terahertz energy, so that terahertz energy with a frequency of 0.1 THz -10 THz may be radiated, and the terahertz energy can penetrate the normal tissues of the human body and reach the lesion site directly, thus accelerating the repair of the lesion site and improving the curative effect.

At least one of the treatment needle 13, the pen cap 12 and the pen rod 11 has terahertz energy, specifically, any one of the treatment needle 13, the pen cap 12 and the pen rod 11 may have terahertz energy, or any two of the treatment needle 13, the pen cap 12 and the pen rod 11 may have terahertz energy, or the treatment needle 13, or the treatment needle 13, the pen cap 12 and the pen rod 11 all have terahertz energy at the same time. Preferably, the treatment needle 13, the pen cap 12 and the pen rod 11 have terahertz energy at the same time. In this way, terahertz energy may be continuously and stably radiated in the treatment process, and more terahertz energy can be radiated, thus improving the treatment efficiency.

In the treatment pen 1 provided by the present disclosure, when the treatment needle 13, the pen cap 12 or the pen rod 11 have terahertz energy, the material of the treatment needle 13, the pen cap 12 or the pen rod 11 should be a material that can store terahertz energy and meet the medical standards. For example, it may be a medical metal material, preferably medical stainless steel.

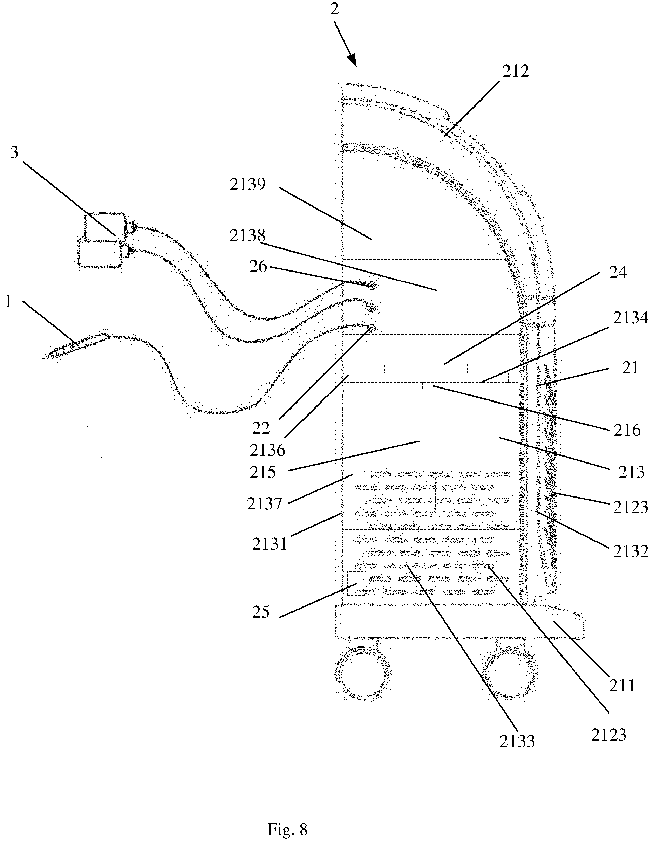

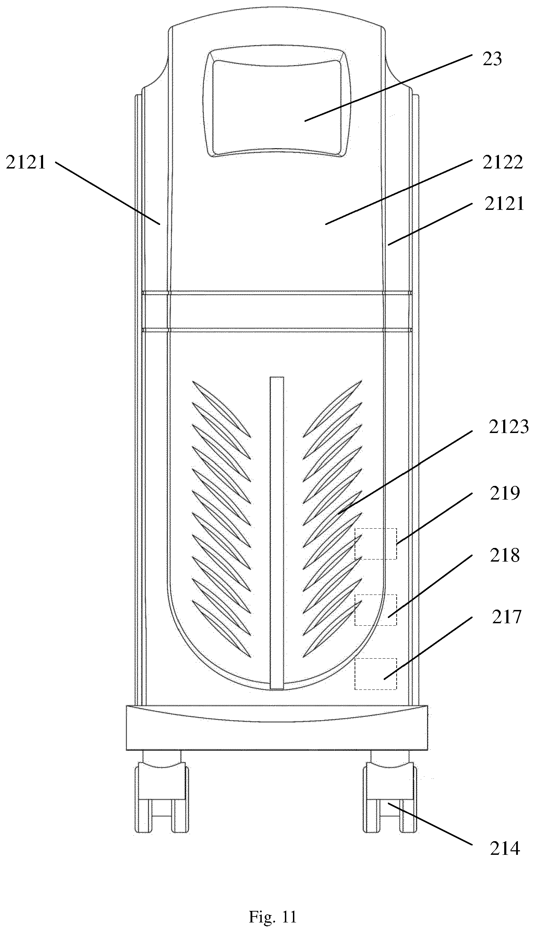

As shown in Fig. 8 and Fig. 11, an embodiment of the present disclosure also provides a main machine for a limb pain treatment instrument. The main machine 2 includes a chassis 21, which comprises a touch display screen 23 arranged on a surface of the chassis 21, and a CPU (Central Processing Unit) main control board 24 arranged in the chassis 21 and electrically connected to the touch display screen 23. The CPU main control board 24 is used for outputting a vibration prescription signal to the vibrator 15, and the touch display screen 23 is used for a user to select a vibration prescription, wherein the vibration prescription signal is output to the treatment pen described in any of the above embodiments.

The shape of the chassis 21 of the main machine 2 may be cuboid, cube or a chassis 21 composed of a plurality of different flat plates connected by welding or fasteners, which can contain articles. A treatment pen output interface 22 may be arranged on the surface of the chassis 21, which is used to transmit vibration prescription signals. Therefore, the surface of the chassis 21 is provided with the treatment pen output interface 22 penetrating through the surface of the chassis, and the treatment pen output interface 22 may be electrically connected to the treatment pen 1 through a cable, specifically, to the vibrator 15 in the treatment pen 1. In other embodiments of the present disclosure, the treatment pen output interface 22 may also adopt a hidden design on the surface of the chassis 21. For example, the surface of the chassis 21 is provided with a detachable rubber plug. After the rubber plug is detached, the treatment pen output interface 22 is exposed. When the treatment pen output interface 22 is not in use, the rubber plug may be better waterproof and dustproof.

The CPU main control board 24 is arranged in the chassis 21 of the main machine 2, and the CPU main control board 24 is electrically connected to the treatment pen output interface 22 and the touch display screen 23, respectively, which can be specifically connected by cables. The CPU main control board 24 is electrically connected to the treatment pen output interface 22, and the treatment pen output interface 22 is electrically connected to the treatment pen 1. Therefore, the CPU main control board 24 can transmit the vibration prescription signal to the treatment pen 1 and the vibrator 15 through the treatment pen output interface 22 to supply power to the vibrator 15 and output the vibration prescription signal. It should be noted here that the cable electrically connected between the treatment pen 1 and the main machine 2 may include a plurality of wires, some of which may be used for the main machine 2 to supply power to the treatment pen 1, and some of which may be used for the main machine 2 to transmit vibration prescription signals to the treatment pen 1.

The surface of the chassis 21 of the main machine 2 is also provided with a physical entity switch (for example, a push-type entity switch or a toggle-type entity switch), which may control the start and stop of the main machine 2. When the user operates the physical entity switch, the main machine 2 is started and the touch display screen 23 is turned on. At this time, the user may click the vibration prescription selection button on the touch display screen 23 to select a corresponding vibration prescription for the treatment site. Then, after clicking the running button, the CPU main control board 24 transmits the vibration prescription signal corresponding to the selected vibration prescription to the treatment pen 1. The treatment pen 1 receives the vibration prescription signal. Next, the user operates the switch button 14 on the treatment pen 1, and the switch button 14 is turned on, and the vibrator 15 starts to generate physical kinetic energy. In addition, the limb pain treatment instrument may also include a remote controller, which is similar to some or all functions of the touch display screen 23, and may control the start and stop of the main machine 2 and the treatment pen 1, specifically, it may control the start and stop of the main machine 2 and the treatment pen 1 by using the infrared technology. The remote controller may make the operation more convenient.

The main machine 2 may be supplied power by commercial power. In this case, the main machine 2 further includes a power supply module 25 arranged in the chassis 21, which is electrically connected to the CPU main control board 24 and the touch display screen 23, respectively. The power supply module 25 is, for example, a power supply adapter, which is used to convert external 220V AC into 13V or less DC.

And/or, the main machine 2 may further include a second power supply device arranged in the chassis 21 and electrically connected to the CPU main control board 24 and the touch display screen 23, respectively, and the second power supply device includes an UPS (uninterruptible power supply), a storage battery or a rechargeable battery. In this way, when the commercial power is cut off, the second power supply device may be selected to continue supplying power to the main machine 2. In addition, when the use environment of the limb pain treatment instrument is inconvenient to be powered by the commercial power, the second power supply device may also be selected to supply power to the main machine 2.

In some embodiments of the present disclosure, as shown in Fig. 8, the chassis 21 of the main machine 2 includes a base 211, a top bracket 212, and a side wall assembly 213 installed between the base 211 and the top bracket 212. The base 211, the top bracket 212, and the side wall assembly 213 enclose a closed structure, and the treatment pen output interface 22 is disposed on the surface of the side wall assembly 213. As shown in Fig. 11, the top bracket 212 includes two opposite upright plates 2121 and an arc-shaped plate 2122 located between the two upright plates 2121, and the touch display screen 23 is fixed on the surface of the arc-shaped plate 2122. At least three casters 214 are provided at the bottom of the chassis 21.

The base 211 may be a flat plate structure, or a cubic structure in the shape of a cube or a cuboid with a plurality of flat plates fixedly connected to each other. At least three casters 214 are arranged at the bottom of the chassis 21, specifically, at least three casters 214 may be arranged at the bottom of the base 211, in order to facilitate the movement of the main machine 2, the casters 214 may be universal wheels, and at least one of the casters 214 has a braking function.

The top bracket 212 includes two opposite upright plates 2121 and an arc-shaped plate 2122 located between the two upright plates 2121, wherein the arc-shaped plate 2122 is bent toward the outside of the chassis 21, and the bending extension direction of the arc-shaped plate 2122 is the length direction of the main machine 2, and the touch display screen 23 may be fixed on the surface of the arc-shaped plate 2122, so that it is convenient for medical practitioners of related profession to operate the touch display screen 23.

The base 211, the top bracket 212, and the side wall assembly 213 may be enclosed into a closed structure, so that the assemblies installed inside the chassis 21 can be protected and dustproof.

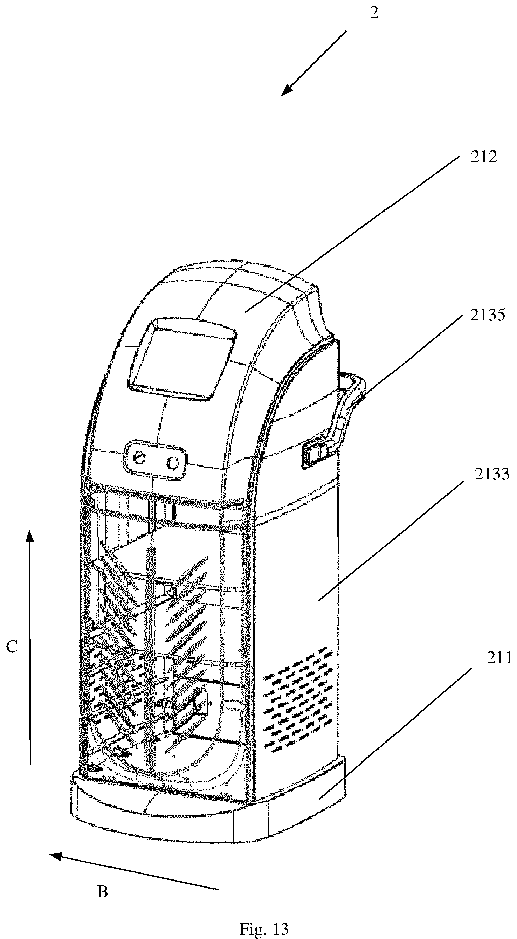

In some embodiments, as shown in Fig. 8, the side wall assembly 213 includes a rear cover plate 2131, a front plate 2132 opposite to the rear cover plate 2131, and two side brackets 2133 disposed between the rear cover plate 2131 and the front plate 2132, a mounting plate 2134 is connected between the two side brackets, and the CPU main control board 24 is fixed on the mounting plate 2134. As shown in Fig. 13, a U-shaped handle 2135 which may freely rotate in a certain angle range is pivotally arranged on outer sides of the two side brackets 2133. The u-shaped handle 2135 may be placed on the back side of the main body 2 when not in use.

The top bracket 212 is fixedly connected to the front plate 2132 and the side bracket 2133 respectively, and the top bracket 212 is detachably connected to the rear cover plate 2131. The base 211 is fixedly connected to the front plate 2132 and the side bracket 2133 respectively, and the base 211 is rotatably connected to the rear cover plate 2131. Here, two assemblies that are fixedly connected may be connected by welding or fasteners.

A mounting plate 2134 is connected between the two side brackets 2133, and the CPU main control board 24 may be fixed on the mounting plate 2134. A U-shaped handle 2135 is pivotally arranged on outer sides of the side brackets 2133. Specifically, one U-shaped handle 2135 can be pivotally arranged on outer sides of the two side brackets 2133, that is, both ends of the U-shaped handle 2135 are pivotally arranged on the same side bracket 2133; or one U-shaped handle 2135 may also be pivotally arranged on two side brackets 2133, that is, one end of the U-shaped handle 2135 is pivotally arranged on one side bracket 2133 and the other end is pivotally arranged on the other side bracket 2133. The U-shaped handle 2135 pivotally arranged on the side brackets 2133 may facilitate medical practitioners of related profession to move the main machine 2.

In some embodiments, as shown in Fig. 8, opposite surfaces of the two side brackets 2133 are both provided with sliding grooves 2136, and the mounting plate 2134 is slidably connected in the sliding grooves 2136. The side bracket 2133 may be a flat plate, and a sliding groove 2136 may be arranged inside the flat plate. The sliding grooves 2136 arranged on the two flat plates correspond in position to each other, and the mounting plate 2134 is slidably connected in the sliding grooves 2136. Since the CPU main control board 24 is fixedly connected on the mounting plate 2134, when the CPU main control board 24 needs to be disassembled, replaced or repaired due to failure, the mounting plate 2134 may slide along the sliding groove 2136 and be removed from the chassis 21, making it easier to remove and replace the CPU main control board 24.

In some embodiments, as shown in Fig. 8, the side bracket 2133 includes an outer side plate 2137, a plurality of first rib plates 2138 fixedly connected to an inner side of the outer side plate 2137 and arranged along the height direction of the outer side plate 2137, and a plurality of second rib plates 2139 arranged along the width direction of the outer side plate 2137, wherein the sliding groove 2136 is formed between two adjacent second rib plates, the angle between a first rib plate and a second rib plate is 90°. The outer plate 2137 may be a flat plate or a U-shaped plate connected to the front plate 2132 and the rear cover plate 2131, respectively. With the above structure, the strength between the side parts may be increased, thereby increasing the strength of the whole chassis 21, and reducing the depression caused by the impact or extrusion of the chassis 21.

It should be noted that with reference to Fig. 13, the "width" direction refers to the direction shown by B, and the "height" direction refers to the direction shown by C.

In some embodiments, as shown in Figs. 8 and 11, the two side brackets 2133 and the front plate 2132 are both provided with a plurality of heat dissipation holes 2123, and the heat dissipation holes 2123 at least correspond to the position of the CPU main control board 24. In this way, the CPU main control board 24 can dissipate heat in time.

In some embodiments, a receiving box 215 is arranged below the mounting plate 2134. Specifically, there are multiple sliding grooves 2136 provided on the side bracket 2133 so that the receiving box 215 may be slidably attached in the sliding grooves 2136 located below the mounting plate 2134. It should be noted that the receiving box 215 may be arranged inside the chassis 21 without extending out of the back cover 2131 or the front plate 2132. When the receiving box 215 is taken out for use, the receiving box 215 may be taken out by detaching the back cover 2131 or the front plate 2132. Alternatively, the front plate 2132 may be provided with an protrusion hole through which the receiving box 215 extends out of the front plate 2132, so that it is convenient to take out and install the receiving box 215, and thus it is convenient to place items in or take out items from the receiving box 215. The drawer-type design makes it more convenient to disinfect the items contained in the receiving box 215 of the main machine 2.

The bottom of the mounting plate 2134 is provided with an ultraviolet disinfection lamp 216, and the ultraviolet disinfection lamp 216 is used for ultraviolet disinfection of the items placed in the receiving box 215. Items placed in the receiving box 215 may include daily medical instruments or supplies such as treatment pen 1, electrode sheet 3, treatment needle 13 or gauze. The ultraviolet disinfection lamp 216 may be fixedly connected to the bottom of the mounting plate 2134, and the light is directed toward the items placed in the receiving box 215, so that the ultraviolet disinfection lamp 216 may disinfect the items placed in the receiving box 215 on a daily basis, and keep the daily medical instruments or supplies clean.

In some embodiments, as shown in Fig. 8, the main machine 2 further includes a power outlet 217 disposed on the surface of the chassis 21, a power fuse 218 disposed in the chassis 21 and electrically connected to the power outlet 217, and a power transformer 219 disposed in the chassis 21 and electrically connected to the power fuse 218. The power transformer 219 is used to supply power to the CPU main control board 24.

In some embodiments, as shown in Fig. 8, the main machine 2 further includes at least one intermediate frequency output interface 26 arranged on the surface of the chassis 21, and the output voltage value of the intermediate frequency output interface 26 is between 0VP-P and 120VP-P.

As shown in Fig. 8, the limb pain treatment instrument further includes at least one group of electrode sheets 3 electrically connected to at least one intermediate frequency output interface 26 via cables respectively, and the CPU main control board 24 is also electrically connected to at least one intermediate frequency output interface 26 for outputting a current prescription signal to at least one group of electrode sheets 3, wherein the current prescription signal may be intermediate frequency current, and one group of electrode sheets 3 may include two electrode sheets 3. As shown in Fig. 5, the output pulse frequency provided by the electrode sheet 3 is between 0.5KHz and 3KHz, the pulse duty ratio is between 10% and 99%, and the error is ±30%. According to electromyography, the main vibration frequency of muscle is about 2KHz. Therefore, the carrier frequency of pulse wave is set between 0.5KHz and 2KHz, which makes the carrier frequency close to the main vibration frequency of muscle. Therefore, the electrode sheet 3 may produce better treatment effect on the lesion site.

Medium frequency current may cause comfortable tremor and muscle tremor, which excites the coarse fibers within the lesion site that mainly conduct tactile pressure sensation and masks the pain sensation conducted by fine and coarse fibers, thus achieving the purpose of pain relief. Medium frequency current may also excite nerve roots, dilate blood vessels, promote blood circulation, improve blood supply to muscles, relax muscle groups, and accelerate the discharge of local pain-causing substances. In addition, when the human body is stimulated by electricity, the nervous system may release some morphine-like substances with analgesic effect, such as endorphin, which can increase the content in nerve tissue, cerebrospinal fluid and even blood plasma, so as to achieve the purpose of analgesia.

Axonal reflex and triple reaction: axonal reflex means that, when electric current acts on the surface of the human body, the electric stimulation passes through the afferent nerve to the posterior horn of the spinal cord, excites the efferent nerve, and causes the small arteries of the skin to expand, resulting in diffuse redness and other symptoms on the skin surface under the electrode sheet 3. Medium frequency current may cause rhythmic contraction and relaxation of muscles, and promote the reflux of blood and lymph. Metabolites of muscle activity, such as lactic acid, ATP, ADP, etc., have obvious vasodilating effect, which may excite neuromuscular, soften scars, loosen induration and adhesion. Medium frequency current may change the ion permeability of cell membrane, leading to the change of polarity inside and outside the cell membrane, depolarizing the membrane potential, forming action potential, thus exciting nerve muscles, producing muscle contraction, exercising muscles and preventing muscle atrophy. Medium frequency current can enlarge the gap between cells and tissues, and loosen the attached connective tissue fibers, muscle fibers and nerve fibers; so as to promoting the repair of the lesion site.

In some embodiments of the present disclosure, the characteristic parameters of the vibration prescription signal include a continuous vibration of the vibrator 15 and a vibration frequency and an amplitude of the continuous vibration, or an intermittent vibration of the vibrator 15 and a duty ratio, a vibration frequency and an amplitude of the intermittent vibration. The vibration mode provided by the CPU control board 18 for the vibrator 15 may be continuous vibration or intermittent vibration, and the specific vibration mode is determined by the vibration prescription signal. Therefore, the characteristic parameters of the vibration prescription signal may include the continuous vibration of the vibrator 15 and the frequency and amplitude of the continuous vibration, or the intermittent vibration of the vibrator 15 and the duty ratio, frequency and amplitude of the intermittent vibration. In addition, the characteristic parameters of the vibration prescription signal may also include the vibration speed. The vibration frequency refers to the number of vibration cycles generated by the vibrator 15 per second; the amplitude represents the maximum vibration displacement of the vibrator 15; the vibration speed represents the effective value of the vibration speed of the vibrator 15 in the vibration process, specifically characterizing the value obtained by the derivative of the vibration amplitude with respect to time; the duty ratio represents that in one vibration cycle, the ratio of the duration of vibration to the total duration in a vibration cycle.

In some embodiments, the vibration prescription includes stored default fixed parameters of each treatment site and an individual vibration intensity level manually adjusted for the same treatment site. Since the muscle sensitivity of different lesion sites is different, a number of different limb vibration intensity levels may be set for different treatment sites, and the default fixed parameters corresponding to different limb vibration intensity levels are different. The default parameter may include the amplitude of the vibrator 15, and the higher the limb vibration intensity level, the greater the amplitude of physical kinetic energy generated by the vibrator 15. Specifically, three different limb vibration intensity levels may be set: strong, medium and weak, in which the muscle sensitivity at shoulder, back, waist, hip, thigh, knee and heel is low, so the limb vibration intensity levels at these parts may be set to strong. The limb vibration intensity level of elbow, wrist and ankle may be set to medium; the muscles in the head, neck, fingers and toes are sensitive, so the limb vibration intensity level in these parts may be set to weak.

In addition, since people of different genders, ages and body types have different muscle sensitivities, different individual vibration intensity levels may be set for different people. For example, three different individual vibration intensity levels may be set: high, medium and low. The higher the individual vibration intensity level, the greater the amplitude of physical kinetic energy generated by the vibrator 15. In this way, it is possible to flexibly select a limb vibration intensity level in order to treat the lesion site of different individuals, and to treat people with different muscle sensitivities in a targeted manner, thus achieving better treatment effect on the basis of minimizing discomfort to human body.

The touch display screen 23 is used for the user to select a corresponding vibration prescription for a treatment site, and is used to generate target characteristic parameters together according to the default fixed parameter included in the selected vibration prescription and the individual vibration intensity level manually adjusted by the user for the same treatment site, and send the target characteristic parameters generated according to the vibration prescription selected by the user to the CPU main control board 24. The touch display screen 23 may display a virtual button for selecting a treatment site and a virtual button for selecting or inputting an individual vibration intensity level, and users may select the treatment site and individual vibration intensity level according to their own requirements.

In the touch display screen 23, a corresponding relationship between lesion sites and individual vibration intensity levels and characteristic parameters may be stored in advance, and the same treatment site and different individual vibration intensity levels may uniquely correspond to a set of characteristic parameters. For different treatment sites, if the corresponding limb vibration intensity level is the same, the same individual vibration intensity level can also correspond to the same characteristic parameters.

After the user selects a treatment site and an individual vibration intensity level in the touch display screen 23, the touch display screen 23 may determine the target characteristic parameters corresponding to the vibration prescription selected by the user according to the treatment site and individual vibration intensity level selected by the user and the corresponding relationship between treatment sites and individual vibration intensity levels and characteristic parameters, and the touch display screen 23 may transmit the target characteristic parameters to the CPU main control board 24.

The main CPU control board 24 is used to generate a corresponding vibration prescription signal according to the target characteristic parameters. After the CPU main control board 24 receives the target characteristic parameters, it may generate the vibration prescription signal corresponding to the vibration prescription input by the user according to the target characteristic parameters, wherein the characteristic parameters in the vibration prescription signal are the target characteristic parameters.

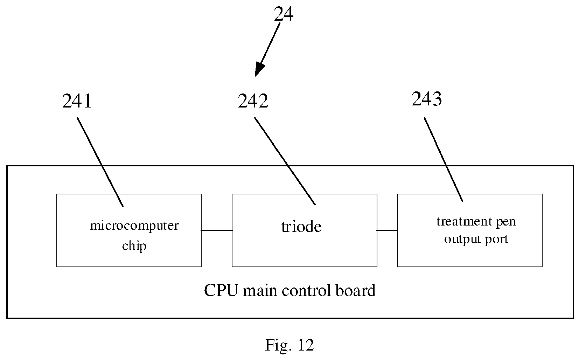

In some embodiments, as shown in Fig. 12, the CPU main control board 24 includes a microcomputer chip 241 and a triode 242. The microcomputer chip 241 is used to receive the target characteristic parameters and determine a target duty ratio according to an amplitude in the target characteristic parameters and a pre-stored correspondence between amplitudes and duty ratios; generate the vibration prescription signal according to the target duty ratio and a vibration frequency in the target characteristic parameters, and send the vibration prescription signal to the triode 242.

Further, the CPU main control board 24 further includes a treatment pen output port 243, which is electrically connected to the treatment pen output interface 22. The microcomputer chip 241 is electrically connected to the triode 242, and the triode 242 is electrically connected to the treatment pen output port 243.

The microcomputer chip 241 includes a PWM (Pulse width modulation) module, which has a storage space to store the corresponding relationship between amplitudes and duty ratios in advance, and the PWM module of the microcomputer chip 241 may receive the target characteristic parameters, and determine the target duty ratio according to the amplitude in the target characteristic parameters and the corresponding relationship between amplitudes and duty ratios. Then, the PWM module may generate the vibration prescription signal according to the target duty ratio and the vibration frequency in the target characteristic parameters, and send the vibration prescription signal to the triode 242. The duty ratio represents the proportion of the power-on time to the total time in a pulse cycle. For example, the duty ratio may be 50%, 60% and other values. It should be noted that the voltage value of the vibration prescription signal is constant. When the duty ratio is different, the powers generated by the vibrator 15 when receiving the vibration prescription signal are different, which may generate different amplitudes. Therefore, the corresponding relationship between the amplitudes and the duty ratios may be stored in advance.

The triode 242 is used to output the vibration prescription signal to the treatment pen 1 and control the vibration prescription signal to drive the vibrator 1. Specifically, the triode 242 is used to output the vibration prescription signal to the treatment pen output port 243 and control the vibration prescription signal to drive the vibrator 15. The function of the triode 242 is to amplify a weak signal into an electrical signal with a larger amplitude, which may usually be used as a contactless switch. Therefore, after receiving a vibration prescription signal, the triode 242 may output the vibration prescription signal to the treatment pen output port 243, and through the action of the triode 242, the vibration prescription signal may drive the vibrator 15 of the treatment pen 1 to generate physical kinetic energy.

In the embodiment of the present disclosure, a memory module, a microprocessor module, a timer and a counter may be integrated inside the touch display screen 23. In other embodiments, the touch display screen 23 is only used for display, and does not have the functions of storage, processing, timing and counting, which are all implemented in the CPU main control board 24. The specific solution of the embodiment of the present disclosure may be as follows:
The touch display screen 23 is used to send the vibration prescription selected by the user to the microcomputer chip 241 of the CPU main control board 24.

The microcomputer chip 241 is used to determine the target characteristic parameters corresponding to the vibration prescription selected by the user according to the stored corresponding relationship between lesion sites, individual vibration intensity levels and characteristic parameters, and the vibration prescription sent by the touch display screen 23; determine the target characteristic parameters corresponding to the vibration prescription selected by the user; determine the target duty ratio according to the amplitude in the target characteristic parameters and the pre-stored correspondence between amplitudes and duty ratios; generate a vibration prescription signal according to the target duty ratio and the vibration frequency in the target characteristic parameters, and send the vibration prescription signal to the triode 242.

The triode 242 is used to output the vibration prescription signal to the treatment pen output port 243 and control the vibration prescription signal to drive the vibrator 15

In some embodiments of the present disclosure, a timer and a counter are integrated in the touch display screen 23, and the timer is used to turn off the main machine 2 or send an alarm when a duration for the main machine 2 outputting vibration prescription signals to the treatment pen 1 reaches a preset duration threshold; the counter is used to record the number of times that the main machine 2 outputs the vibration prescription signal to the treatment pen 1.

For each patient, a maximum treatment duration may be set, i.e., the preset duration threshold, which may be a preset duration, for example, 15 minutes, set by the medical practitioner of related profession, indicating that the treatment duration is no more than 15 minutes for each patient. The touch display screen 23 may also display a timing time, which may be a forward timing from 0 to the preset duration threshold, or a countdown from the preset duration threshold to 0. When a user turns on the physical entity switch of the main machine 2, the timer starts timing. When the treatment duration for the same patient reaches the preset duration threshold, the touch display screen 23 may send an alarm or turn off the main machine 2, so as to avoid the negative impact on the patient caused by the negligence or error of the medical practitioner of related profession.

For the treatment of the same patient, when the physical entity switch of the main machine 2 is turned on, the main machine 2 may continuously output vibration prescription signals to the treatment pen 1. Within the maximum treatment duration of each patient, the medical practitioner of related profession may respectively treat a plurality of different lesion sites of the patient, and the treatment duration of each lesion site is less than 3 minutes.

When medical practitioners of related profession click Start on the touch display screen 23 for different patients, the counter may add one, and the currently counted number is displayed on the touch display screen 23, which indicates the number of times that the main machine 2 outputs the vibration prescription signal to the treatment pen 1, that is, the counter indicates the total number of times the limb pain treatment instrument performs treatment in the embodiment of the present disclosure.

As shown in Figs. 8 and 18, the embodiment of the present disclosure also provides a limb pain treatment instrument, which includes the treatment pen 1 as described in any one of the above embodiments and the main machine 2 as described in any one of the above embodiments, wherein the treatment pen 1 is an example of a controlled device and the main machine 2 is an example of a controlled device.

A touch display screen 23 is arranged on the surface of the chassis 21 of the main machine 2, and the user may select the vibration prescription through the touch display screen 23. It should be noted here that, since the limb pain treatment instrument is a medical instrument, the limb pain treatment instrument is usually operated by a medical practitioner of related profession, that is, the user who inputs the vibration prescription on the touch display screen 23 is a professional medical practitioner of related profession.

The connection mode between the main machine 2 and the treatment pen 1 includes wired connection and/or wireless connection. When the main machine 2 is wirelessly connected to the treatment pen 1, Bluetooth connection, infrared connection or Wi-Fi connection may be adopted. The main machine 2 further includes a first signal transceiving module (not shown) located within the chassis 21 and electrically connected to the CPU main control board 24, and the CPU main control board 24 can output vibration prescription signals to the treatment pen 1 through the first signal transceiving module; the treatment pen 1 further includes a second signal transceiving module for receiving vibration prescription signals, and a first power supply device for supplying power to the vibrator 15, the second signal transceiving module and the switch button 14. The first power supply device may include a button battery, a dry battery or a rechargeable battery.

When the main machine 2 is connected to the treatment pen 1 by wires, the main machine 2 and the treatment pen 1 are electrically connected by cables. Specifically, the surface of the chassis 21 is also provided with a treatment pen output interface 22 electrically connected to the CPU main control board 24. The treatment pen output interface 22 is electrically connected to the treatment pen 1 by cables, and the main machine 2 may supply power and transmit vibration prescription signals to the treatment pen 1 through the treatment pen output interface 22 and cables.

When the limb pain treatment instrument according to the embodiment of the present disclosure is used to treat the lesion site of human body, the physical entity switch set on the main machine 2 is operated by a medical practitioner of related profession, the main machine 2 is started, and the touch display screen 23 is started. The medical practitioner of related profession may select the vibration prescription after operating the touch display screen 23 to log in or set up, and the CPU main control panel 24 may output the vibration prescription signal corresponding to the vibration prescription and transmit the vibration prescription signal to the vibrator 15 of the treatment pen 1 through the treatment pen output interface 22. Then, the medical practitioner of related profession installs the treatment needle 13 on the mounting bracket 17 of the pen rod 11, and after fixing the pen cap 12 on the pen rod 11, presses the timing key "Run" on the touch display screen 23 to insert the treatment needle 13 into the lesion site. Then, by operating the switch button 14, the vibrator 15 is started, the physical kinetic energy is generated according to the vibration prescription signal, and the physical kinetic energy is transmitted to the treatment needle 13, so that the treatment needle 13 brings the physical kinetic energy into the lesion site for treatment. When the treatment of the lesion site is finished, the switch button 14 may be stopped, the vibrator 15 stops vibrating, then the treatment needle 13 is pulled out from the lesion site, the vibration prescription is selected on the touch display screen 23 again, and the above operations are continued until the treatments of all the lesion sites of the patient are finished.

In the embodiment of the present disclosure, the treatment pen 1 can output vibration only when it is controlled by the switch button 14 and the main machine 2 at the same time, that is, a "double switch" design is adopted, which makes the safety of the use of the limb pain treatment instrument higher.

It should be noted that, when the limb pain treatment instrument according to the embodiment of the present disclosure is used to treat the lesion site of the human body, it is also possible for a medical practitioner of related profession to install the treatment needle 13 on the pen cap 12 at first, and then operate the physical entity switch provided on the main machine 2 to start the main machine 2.

In the embodiment of the present disclosure, the limb pain treatment instrument includes a main machine 2 and a treatment pen 1, and the CPU main control board 24 in the main machine 2 may output the vibration prescription signal corresponding to the vibration prescription to the vibrator 15 of the treatment pen 1 through the treatment pen output interface 22. The vibrator 15, when it is started and receives the vibration prescription signal, can generate physical kinetic energy corresponding to the vibration prescription signal, and transmit the physical kinetic energy to the treatment needle 13 installed in the pen rod 11, so that the treatment needle 13 brings the physical kinetic energy into the lesion site while intervening in the lesion site, so that the damaged adhesions and nodules may be better loosened to realize the treatment of the lesion site, and thus achieve the purpose of improving pain or even eliminating the lesion.

In addition, the limb pain treatment instrument provided by some embodiments of the present disclosure can also play a role in diminishing inflammation, relieving pain, relieving muscle fatigue, promoting nerve repair, improving blood circulation, and treating the resulting associated limb pain and discomfort such as bone lesions and joint pain, such as cervical spondylosis, scapulohumeral periarthritis, lumbar spondylosis or arthritis, etc..

As shown in Fig. 8, the limb pain treatment instrument further includes at least one group of electrode sheets 3 each electrically connected to at least one intermediate frequency output interface 26 through cables, and the CPU main control board 24 is also electrically connected to at least one intermediate frequency output interface 26 for outputting current prescription signals to at least one group of electrode sheets 3, wherein the current prescription signals may be intermediate frequency current, and one group of electrode sheets 3 may include two electrode sheets 3. As shown in Fig. 21, the electric shock pulse frequency provided by the electrode sheet 3 is between 0.5KHz and 3KHz, and the pulse width is between 100 µs and 400 µs, the error is 30%.

In some embodiments, the display interface of the touch display screen 23 is provided with a vibration prescription selection button and a running button. The vibration prescription selection button is used for the user to select the corresponding vibration prescription for the treatment site. The CPU main control panel 24 of the main machine 2 may transmit the vibration prescription signal corresponding to the vibration prescription to the treatment pen 1 after the user clicks the running button. The treatment pen 1 controls the vibrator 15 to vibrate according to the vibration prescription signal when the vibration prescription signal is received and the switch button 14 is turned on. The specific presentation mode of the running button on the display interface is not limited, for example, it may be icon, text, etc.. The specific icon style and text content are not limited.

The lesion site may be treated with the limb pain treatment instrument of the embodiment of the present disclosure according to the following steps:
1) Before treatment, ensure that the surface of the patient's lesion site is clean and placed stably, all accessories of the limb pain treatment instrument are firmly connected, the power supply is grounded, and the main machine 2 is stopped or shutdown state or the intensity output is zero.
2) Plugging the power plug of the main machine 2 into the AC 220V or 50Hz power outlet, and turning on the power by pressing the physical entity switch of the main machine 2 to the "-" position. At this time, the touch display screen 23 of the main machine 2 lights up to show the normal power-on.
3) Preparation of electrode sheet 3
4) Electrically connecting the electrode sheet 3 to the main machine 2, and attaching the electrode sheet 3 to the lesion site, the effect is better when it is fully contacted with the skin.
5) Performing the treatment of the lesion site with the treatment pen 1.
   ① Inserting the cable at the proximal end of the pen rod 11 of the treatment pen 1 into the treatment pen output interface 22 of the main machine 2.
   ② The pen cap 12 of the treatment pen 1 is disinfected with alcohol or iodophor for use.
   ③ The treatment needle 13 installed in the treatment pen 1 is selected from a specialized treatment needle or a common disposable syringe needle from a regular manufacturer. The structural form, specification and size of the treatment needle 13 are not specifically limited, and the specific configuration is made according to the depth of the position where muscles need to be loosened.
   ④ First, installing the sterile treatment needle 13 on the mounting bracket 17 of the pen rod 11, then gently mount and fasten the pen cap 12 of the sterilized treatment pen 1, and gently press the switch button 14 to properly adjust the individual vibration intensity level or select the lesion site, and then testing whether the vibration function of the treatment pen 1 is normal, so as to be ready for use.

Pressing the timer "Run" key on the touch display screen 23, inserting the treatment needle 13 into the lesion site, and then pressing the switch button 14 of the pen rod 11 continuously to release and recover the lesion site by means of physical kinetic energy.

An embodiment of the present disclosure provides a vibration testing apparatus for a treatment pen for a limb pain treatment instrument, to perform vibration testing on the treatment pen, and the accuracy, reliability and consistency of repeated detection of the test results are high; furthermore, the convenience of moving and transporting the testing apparatus is high, and the cost is low.

In the embodiment of the present disclosure, it is defined that the end of the treatment pen close to the operator, such as a doctor, is the proximal end of the treatment pen, and the end away from the operator is the distal end of the treatment pen.

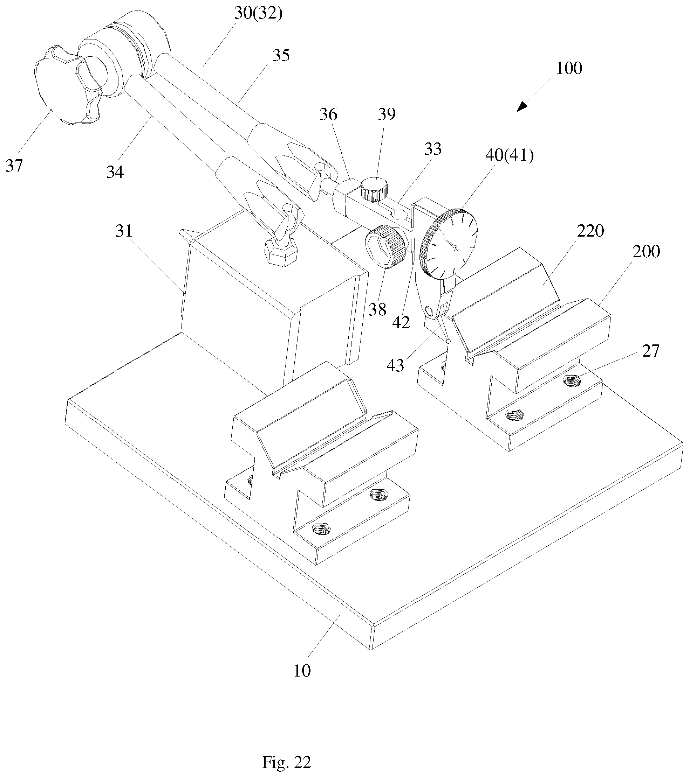

As shown in Fig. 22, Fig. 23a and Fig. 28, a vibration testing apparatus 100 for a treatment pen of a limb pain treatment instrument provided by some embodiments of the present disclosure includes:
a ferromagnet counterweight base 10;
two brackets 200, the top of which has a V-shaped groove 21 extending in the same horizontal direction, the V-shaped groove 210 of each support includes two opposite side walls 220, and each side wall has a supporting convex strip 230 extending along the side wall surface and orthogonal to the horizontal direction, and the support lug 230 of the two brackets 200 are used for supporting the side surfaces of the treatment pen 60;
a magnetic watch base 30, which includes a base body 31 which can be adsorbed and fixed with the ferromagnetic counterweight base 10, and a universal adjusting arm 32 which is connected with the base body 31 by a ball j oint, and the end of the universal adjusting arm 32 is provided with a clamping mechanism 33; and
a lever dial indicator 40, which includes a dial 41, a fixing part 42 clamped and fixed by the clamping mechanism 33, and a measuring meter rod 43 located below the dial 41 and capable of contacting the top of the side surface of the treatment pen 60 on the brackets 200. When the treatment pen 60 vibrates, the reading of the dial 41 displays the corresponding amplitude measurement value.
a power supply device (not shown in the figure) for outputting a plurality of test voltages to the treatment pen.

According to the vibration testing device 100 provided in the embodiment of the present disclosure, the measuring meter rod 43 of the lever dial indicator 40 is in contact with the treatment pen 60. Since the high sensitivity of the lever dial indicator 40, it is easy to capture the subtle changes of the treatment pen 60 when vibrating, and the contact area between the treatment pen 60 and the supporting convex strip 230 on the bracket 200 is small, so that the accuracy of the obtained test results is high. In addition, the vibration testing apparatus 100 provided by the present disclosure has simple structure, no special requirements for the testing environment, less land occupation and low cost; and has high mobility and application flexibility.

The V-shaped groove 210 in the vibration testing apparatus 100 provided by the embodiment of the present disclosure is suitable for treatment pens with different diameters, so that the vibration testing apparatus can test treatment pens with various design specifications, and it is movable and has high application flexibility.

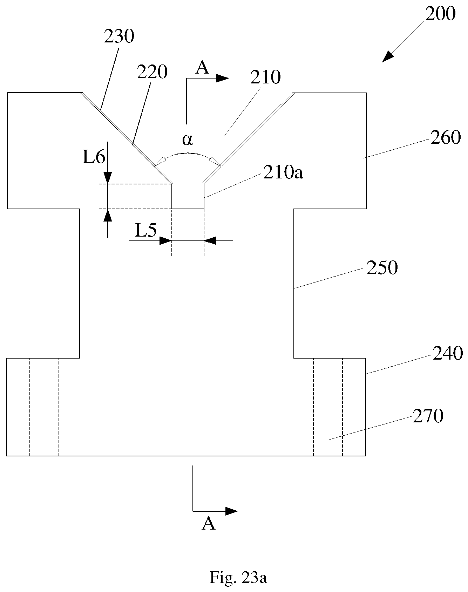

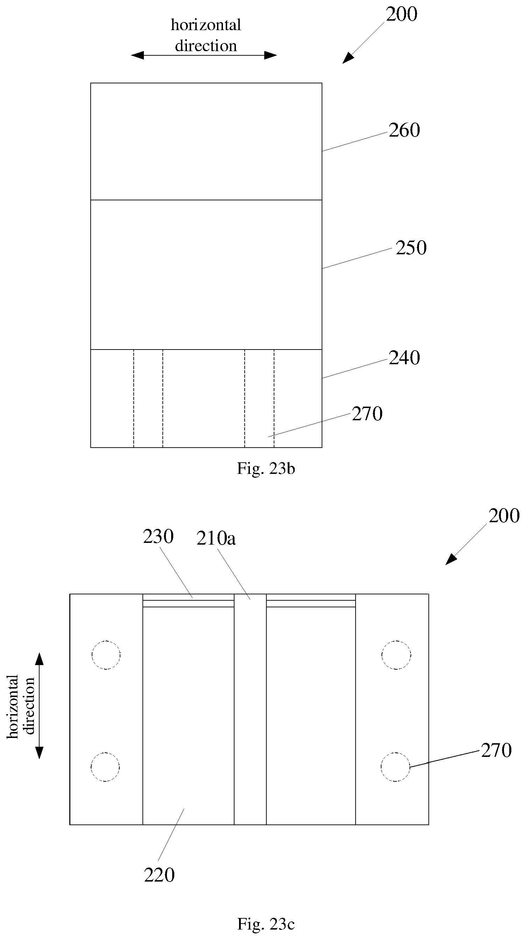

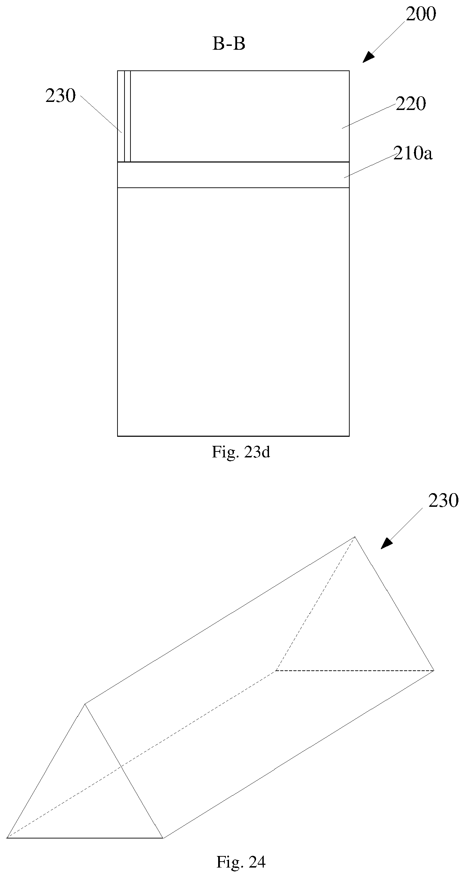

As shown in Fig. 23a, Fig. 23b, Fig. 23c and Fig. 23d, in one embodiment of the present disclosure, each bracket is an I-shaped bracket, including a fixing part 240, a connecting part 250 and a supporting part 260 which are sequentially arranged along the ferromagnetic counterweight base 10. The V-shaped groove 210 is located in the supporting part 260, and the fixing part 240 has a plurality of threaded holes 270, which are connected to the ferromagnetic counterweight base 10 by a plurality of bolt assemblies (not shown in the figure) arranged in the plurality of threaded holes. The I-shaped bracket is used to support the treatment pen 60 with good stability, and it is fixedly connected to the ferromagnetic counterweight base 10 by bolts, which can fix the both firmly, and avoid absorbing the vibration generated by the treatment pen 60 during the testing process due to a gap between the two. The number of screw holes 270 on the fixing part 240 is not particularly limited as long as the purpose of the present disclosure can be achieved. For example, as shown in Fig. 23c, the number of threaded holes 270 is four, and two are distributed on each side. Of course, the number of screw holes 270 on both sides of the fixing part 240 may be the same or different.

In the vibration testing apparatus 100 provided by the present disclosure, in order to avoid the resonance of the bracket 200 in the vibration process of the treatment pen 60, the material for making the bracket 200 can be a metal material with large mass and easy processing, such as iron.

In an embodiment of the present disclosure, the cross section of the supporting convex strip 230 is generally triangular, which means that the cross section is triangular within a certain shape error range, for example, certain rounded corners or oblique chamfers are allowed. As shown in Fig. 24, the cross section of the supporting convex strip 230 may be a conventional triangle, that is, the supporting convex strip 230 is a triangular prism, and one edge of the triangular prism is in contact with the treatment pen 60. Thus, a point contact is formed between the triangular prism and the treatment pen 60. Of course, the cross section of the supporting convex strip may also be at least one corner of a triangle is rounded, and one edge corresponding to the rounded corner contacts with the treatment pen, forming a point contact between the supporting convex strip and the treatment pen. It may be understood that in the process of testing the treatment pen, the smaller the contact area between the supporting convex strip and the treatment pen, the more accurate the test result of the vibration amplitude of the treatment pen, thus improving the accuracy of the test.

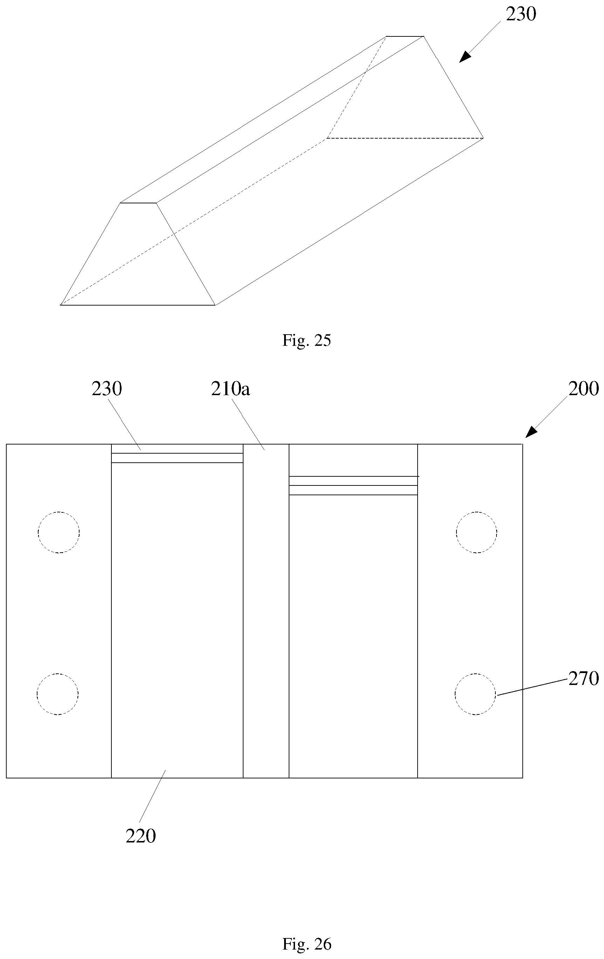

**In** another embodiment of the present disclosure, the cross-section of the supporting convex strip 230 is generally trapezoidal, which means that the cross-section is trapezoidal within a certain error range. As shown in Fig. 25, the cross section of the supporting convex strip 230 may be a conventional trapezoid, or the cross section of the supporting convex strip may be a trapezoid with at least one corner is rounded, wherein the upper bottom of the trapezoid is in contact with the treatment pen 60. Specifically, the upper bottom of the trapezoid is far away from the side wall surface and has a width of 0.1mm to 1.0mm. Within the aforementioned range, not only can the contact between the treatment pen and the supporting convex strip be stable, but also the contact area between the supporting convex strip and the treatment pen is small, which can also improve the accuracy of the test.

As shown in Fig. 23c, in one embodiment of the present disclosure, two supporting convex strips 230 on at least one bracket are arranged in the same longitudinal section, that is, the projections of the two supporting convex strips 230 on the ferromagnetic counterweight base 10 extend to both ends in the same direction.

As shown in Fig. 26, the two supporting convex strips 230 on at least one bracket are staggered in the horizontal direction, which may also support the treatment pen.

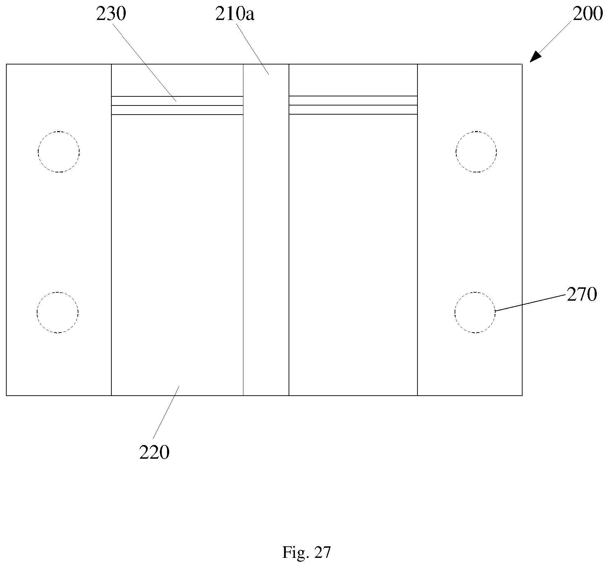

As shown in Fig. 23c, the position of the supporting convex strip 230 on the side wall 220 of the V-shaped groove 210 may be close to the edge of the side wall 220. As shown in Fig. 27, the supporting convex strips 230 may also be provided in the side wall 220 as long as they can support the treatment pen 60.

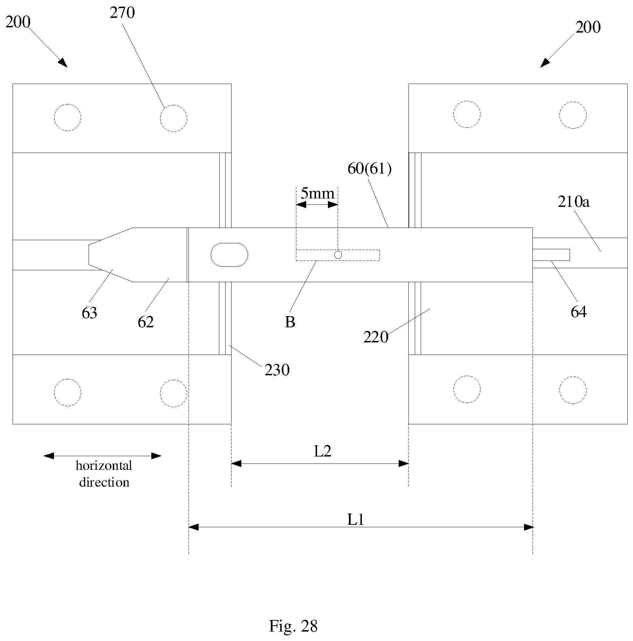

As shown in Fig. 28, specifically, in one embodiment of the present disclosure, the treatment pen 60 includes a pen rod 61, a cap 62 at the distal end of the pen rod 61, and a lead wire 64 at the proximal end of the pen rod 61. The diameter of the pen rod 61 is equal everywhere, and the pen cap 62 includes a pen tip portion 63 whose diameter gradually decreases from near to far. In the horizontal direction, the spacing between the two supporting convex strips 230 located at the two ends of the two brackets 200 is not greater than the length of the pen rod portion, and the lead wire 64 is used to connect with the power supply device, so that that contact positions of the treatment pen 60 and the supporting convex strips 230 all fall on the pen rod 61, and the measured vibration accuracy is higher since the pen rod 61 has a uniform diameter and an integrated structure. The distance between the two supporting convex strips on the two brackets refers to the distance in the horizontal direction between the parts where the supporting convex strips contact with the treatment pen.

As shown in Fig. 28, when the supporting convex strips 230 are arranged at the edges of the side walls 220, the spacing distance between the two supporting convex strips 230 located at both ends of the arrangement of the two brackets 200 is the spacing distance between the two brackets 200. At this time, the distance L2 set between the two brackets 200 should not be greater than the pen rod length L1.

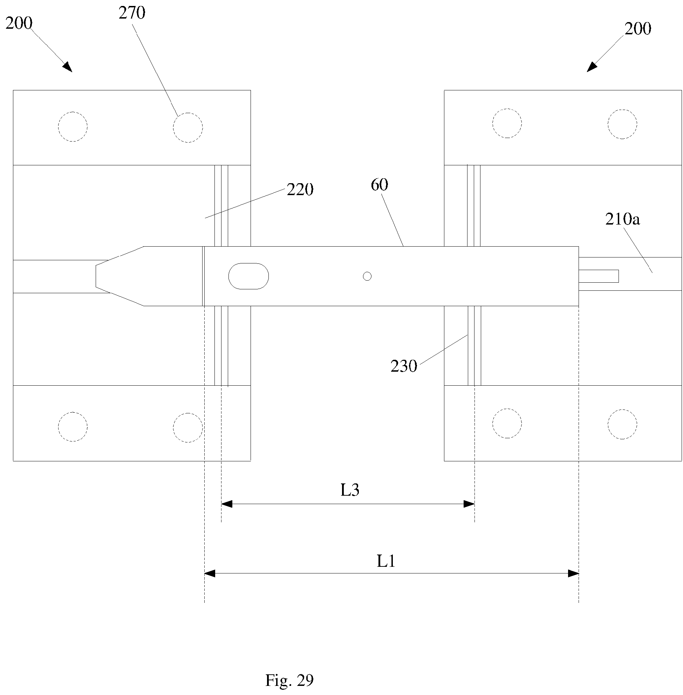

As shown in Fig. 29, when the supporting convex strips 230 are arranged in the side wall 220 and the two supporting convex strips 230 are in the same longitudinal section, the spacing distance between the two supporting convex strips 230 located at both ends of the arrangement of the two brackets 200 is the distance between the supporting convex strips 230 on the two brackets 200. At this time, the distance L3 between the supporting convex strips 230 on the two brackets 200 should not be greater than the pen rod length L1.

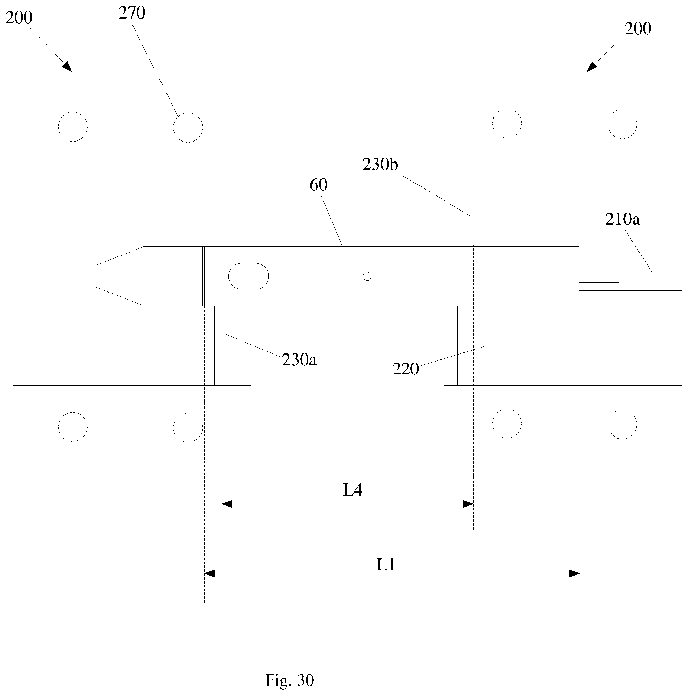

As shown in Fig. 30, when the supporting convex strips 230 are arranged in the side wall 220 and the two supporting convex strips 230 are staggered in the horizontal direction, the spacing distance between the two supporting convex strips 230 located at both ends of the arrangement of the two brackets 200 is the distance between the two supporting convex strips with the longest spacing. At this time, the distance L4 between the supporting convex strips 230a, 230b with the longest spacing on the two brackets 200 should not be greater than the pen rod length L1.

As shown in Fig. 23a, in one embodiment of the present disclosure, the included angle α of the two side walls is 50° to 135°, preferably 75° to 120°, which may effectively support and position the treatment pen.

In an embodiment of the present disclosure, the roughness of the side wall surface of the V-shaped groove is not greater than 1.6µm, so that the vibration of the treatment pen may be prevented from being influenced by excessive roughness, thereby improving the detection accuracy and preventing the surface of the treatment pen from being scratched by vibration.

As shown in Fig. 23a, in one embodiment of the present disclosure, the bottom of the V-shaped groove 210 is provided with a rectangular process groove 210a, and the width L5 of the rectangular process groove 210a is 3mm to 10mm, and the depth L6 is 5mm to 10mm, which facilitates the processing of both side walls of the V-shaped groove.

The lever dial indicator in the vibration testing apparatus 100 provided by the embodiment of the present disclosure is a standard dial indicator in the field, and further includes a transmission mechanism fixed on the back of the dial 41, and the fixing column 42 and the measuring meter rod 43 are arranged on the transmission mechanism. The precision of the lever dial indicator 40 is not more than 0.002mm, and the accuracy of the measurement result is high.

As shown in Fig. 22, in one embodiment of the present disclosure, the universal adjusting arm 32 includes:
a first arm section 34 which is connected to the base 31 by a ball joint and capable of locking the relative position with the base 31, a second arm section 35 which is connected to the first arm section 34 by a hinge joint and capable of locking the relative position with the first arm section 33, and a third arm section 36 which is connected to the second arm section 35 by a ball joint and capable of locking the relative position with the second arm section 34, wherein:
the hinge joint of the first arm section 34 and the second arm section 35 is provided with a bracket adjusting knob 37 for fixing the angle between the first arm section 34 and the second arm section 35; the end of the third arm section 36 is provided with a clamping mechanism 33, which includes two clamping parts arranged opposite to each other, and a fixing adjustment knob 38 for fixing the fixing column 42 of the lever dial indicator 40 between the two clamping parts. The third arm section 36 further includes a fine adjustment knob 39 for fixing the angle and position between the third arm section 36 and the first arm section 34.

The universal adjusting arm 32 is used to adjust and control the position and angle of the lever dial indicator 40 to adapt to different test situations, wherein the ball joint of each arm segment further includes a universal support pad, which may keep the adjusted angle and position unchanged.

In the vibration testing device 100 provided by the present disclosure, the ferromagnetic counterweight base 10 should possess a ferromagnetic body, so that the base 31 of the magnetic watch base 30 may be firmly adsorbed and fixed on the ferromagnetic counterweight base 10, and thus, the universal adjusting arm 32 is not easily to shake under external forces, which will affect the shaking of the lever dial indicator 40.

In the vibration testing apparatus 100 provided by the present disclosure, the power supply device may be the main machine of a limb pain treatment instrument that controls the vibration of a treatment pen, and the main machine may output test modes of different voltages; it may also be a power supply that may output the same test voltage as the main machine. For example, the power supply may be a multi-stage adjustable DC power supply.

After the treatment pen 60 is manufactured, measuring the vibration amplitude of the treatment pen 60 under different input voltages to determine whether it meets the product design requirements and factory quality standards. Testing with the above vibration testing apparatus 100 includes the following steps:
placing the treatment pen 60 in the V-shaped grooves 210 of the two brackets 200, so that the side surfaces of the treatment pen 60 are supported by the supporting convex strips 230 on the two brackets 200;
fixing the magnetic meter base 30, installing the lever dial indicator 40, adjusting the angle and position of the universal adjusting arm 32 and the measuring meter rod 43 of the lever dial indicator 40, and making the measuring meter rod 43 contacting with the top of the side surface of the treatment pen 60;
turning on the power supply device and reading the measured values of the lever dial indicator 40 according to the order of the test voltage increasing or decreasing; when measured values corresponding to respective test voltages are within the corresponding preset range, the test result of the treatment pen is qualified; when a measured value corresponding to at least one test voltage is beyond the corresponding preset range, the test result of the treatment pen is unqualified. The corresponding preset range refers to the effective amplitude range corresponding to each test voltage.

In one embodiment of the present disclosure, after adjusting the angle and position of the measuring rod of the lever dial indicator, and before turning on the power supply device, the following step is further included: zeroing the lever dial indicator so that the reading of the adjusted lever dial indicator is 0.03mm to 0.15mm. The treatment pen is in a stationary state when it is not powered on, and the measuring meter rod 43 may be adjusted to contact with the top of the side surface of the treatment pen 60. When the treatment pen 60 is powered on and starts to vibrate, the contact point of the measuring meter rod 43 contacting the side surface of the treatment pen 60 may change and not located on the top of the side surface of the treatment pen, which may lead to inaccurate reading of the lever dial indicator. Therefore, performing the above steps before the treatment pen 60 starts to be powered on may improve the accuracy of measurement. For example, after zeroing the lever dial indicator, the reading of the lever dial indicator is adjusted to 0.06 mm.

As shown in Fig. 28, in one embodiment of the present disclosure, placing the treatment pen 60 in the V-shaped grooves 210 of two brackets includes: the contact point of the measuring meter rod 43 contacting the treatment pen 60 is located in the range B where the middle point 61 of the treatment pen 60 extends 5mm to both sides along the length direction of the treatment pen. The measuring contact point is located in the middle of the treatment pen, so that the stress is more balanced, and the measured amplitude value may better reflect the working state of the treatment pen, thus the accuracy of the test result is higher.

In an embodiment of the present disclosure, adjusting the angle and position of the measuring meter rod 43 of the lever dial indicator 40 includes adjusting the included angle between the measuring meter rod 43 and the surface of the ferromagnetic counterweight base 10 to 10 to 60 degrees, and within the aforementioned angle range, the measurement result is more accurate, preferably 20 to 50 degrees, and the accuracy of the measurement results may be further improved within the aforementioned preferred angle range.

In an embodiment of the present disclosure, placing the treatment pen 60 in the V-shaped grooves 210 of two brackets 200 includes that the difference of the lengths of the treatment pen 60 on the two brackets 200 is not more than 5mm. So that the stress of the treatment pen on the two brackets is more balanced, and the measured value can better reflect the working state of the treatment pen 60.

Specifically, a treatment pen vibration test method is as follows:
placing the treatment pen on two brackets, and adjusting the positions of the brackets so that the supporting convex strips contact with the pen rod of the treatment pen. Fixing the lever dial indicator by the clamping structure of the universal adjusting arm, and tightening the fixing adjusting knob; adjusting the angle and position of the first arm section and the second arm section so that the third arm section is located in the middle above the two brackets, then tightening the bracket adjustment knob and turning the third arm section so that the measuring rod of the lever dial indicator is located above the middle of the treatment pen, and the angle between the measuring rod and the surface of the ferromagnetic counterweight base is 30 degree. Then, adjusting the fine-tuning knob so that the measuring rod contacts the apex of the side surface of the middle point of the treatment pen. Turning on the DC power supply, measuring the vibration displacements at 2.5V, 3.2V and 4.2V in turn, and recording the values. Comparing the measured values with the preset range of vibration displacements under the corresponding voltages. If all the measured values are within the preset range, the test result of the treatment pen is qualified, otherwise it is unqualified.

It should be noted that in this text, relational terms such as first and second are only used to distinguish one entity or operation from another entity or operation, and do not necessarily require or imply any such actual relationship or sequence between these entities or operations. Moreover, the terms "including", "comprising" or any other variation thereof are intended to cover non-exclusive inclusion, so that a process, method, article or equipment that includes a series of elements not only include those elements, but further include other elements not explicitly listed, or further include elements inherent to such process, method, article or equipment. Without further restrictions, the element defined by the statement 'including one ..." does not exclude the existence of another identical element in the process, method, article or equipment that includes the said element.

All the embodiments in this specification are described in relevant ways, and the same and similar parts of each embodiment can be referred to each other, and the differences between each embodiment and other embodiments are highlighted. Especially, for the method embodiment, the device embodiment, the device embodiment, the computer readable storage medium embodiment and the computer program product embodiment, because they are basically similar to the system embodiment, the description is relatively simple, so please refer to the partial description of the system embodiment.

## Claims

1. A treatment pen (1) for a limb pain treatment instrument comprising a main machine (2) that outputs a vibration prescription signal to the treatment pen (1), wherein the treatment pen (1) comprises:
a pen rod (11) and a pen cap (12) detachably connected to a distal end of the pen rod (11), a treatment needle (13) is mounted in the pen rod (11) after the pen cap (12) is detached, a switch button (14) is provided on a surface of the pen rod (11); a needle tube (131) of the treatment needle (13) extends out of a distal end of the pen cap (12), a vibrator (15) that can be controlled by the switch button (14) to start and stop is provided inside the pen rod (11); the vibrator (15) is configured to, when started, transmit physical kinetic energy corresponding to a vibration prescription signal to the treatment needle (13) intervened into a lesion site,
**characterized in that** an elastic wave bead (16) for mounting and releasing the pen cap (12) is provided at the distal end of the pen rod (11); a contact surface (121) is provided within the pen cap (12), and the contact surface (121) is located at the distal end of the pen cap (12); and the treatment needle (13) also comprises a needle tube holder (132) fixedly connected to the needle tube (131), and when the treatment needle (13) is fixed on the pen rod (11), a distal end surface of the needle tube holder (132) contacts with the contact surface (121).

2. The treatment pen (1) according to claim 1, wherein the needle tube holder (132) comprises a needle tube fixing part (1321) and a clamping part (1322) fixedly connected to the needle tube fixing part (1321), wherein the needle tube fixing part (1321) is provided with a needle tube mounting hole (1323) penetrating through the needle tube fixing part (1321), the needle tube (131) is inserted and fixed within the needle tube mounting hole (1323), the clamping part (1322) is annular and hollow, and a cavity of the clamping part (1322) is communicated with the needle tube mounting hole (1323); the distal end of the pen cap (12) is provided with a needle tube exit hole (122), and the needle tube (131) can pass through the needle tube exit hole (122) and extend out of the distal end of the pen cap (12);
the treatment pen (1) also comprises a mounting bracket (17) provided inside the pen rod (11), wherein the mounting bracket (17) comprises a bracket body (171) extending along a length direction of the pen rod (11) and a mastoid (172) fixedly connected to the distal end of the bracket body (171); and the clamping part (1322) is sleeved and fixed on the mastoid (172);
preferably, wherein the bracket body (171) comprises a first body part (1711) and
a second body part (1712) which are oppositely arranged and fixedly connected, and the vibrator (15) is fixedly connected between the first body part (1711) and the second body part (1712);
the mounting bracket (17) further comprises a side wall (173) fixed to distal ends of the first body part (1711) and the second body part (1712) and surrounding the mastoid (172), and the side wall (173) is in contact with an outer side surface of the needle tube holder (132).

3. The treatment pen (1) according to claim 1, wherein the pen cap (12) is provided with at least one sight window (123) along a circumferential direction, and the needle tube holder (132) is a needle tube holder made of transparent material, and the needle tube holder (132) can be observed through each sight window (123).

4. The treatment pen (1) according to claim 1, wherein a printed circuit board PCB control board (18) is also arranged inside the pen rod (11), an LED backlight (181), a vibration indicator lamp (182) and the switch button (14) are integrated on the PCB control board (18), the LED backlight (181) and the switch button (14) are exposed on a surface of the pen rod (11), and a working observation hole is also arranged on the surface of the pen rod (11), the working observation hole corresponds to a position of the vibration indicator lamp (182), and the LED backlight (181) is closer to the distal end of the pen rod (11) than the vibration indicator lamp (182), the PCB control board (18) is also electrically connected to the vibrator (15).

5. The treatment pen (1) according to claim 1, wherein the treatment pen (1) has at least one of the following characteristics: (a) a trace of vibration output by the vibrator (15) is in a plane perpendicular or parallel to an axis of the pen rod (11), and the trace of the vibration comprises a trace of reciprocating motion in a single direction, and/or a trace of reciprocating motion in multiple angular directions cyclically centred on the axis of the pen rod (11); (b) an amplitude of the vibrator (15) is designed between 2µm and 500µm, and a vibration frequency of the vibrator (15) is designed between 5Hz and 500Hz; (c) the treatment needle (13) is a specialized treatment needle or a disposable syringe needle, which is used to intervene in the lesion site caused by a soft tissue damage for diagnosis and/or treatment.

6. The treatment pen (1) according to claim 1, wherein, the treatment pen (1) further comprises a negative pressure pump (19) detachably connected to the distal end of the pen rod (11); the pen cap (12) is a pen cap made of transparent material; a joint (124) is fixed on a surface of the pen cap (12); the negative pressure pump (19) is connected to the joint (124) through a drainage tube (191); the joint (124) is communicated with the pen cap (12) and the treatment needle (13); and the negative pressure pump (19) is used for providing suction negative pressure for effusion within the lesion site when being started, so that the effusion can be pumped out from the lesion site.

7. The treatment pen (1) according to claim 6, wherein the treatment pen (1) further comprises a communication tube (192) communicating the drainage tube (191) and the joint (124); a control signal for starting and stopping the negative pressure pump (19) comes from the main machine (2), and the main machine (2) generates a corresponding control signal by detecting whether there is effusion at a position where a sensor (193) in the communication tube (192) is located.

8. The treatment pen (1) according to claim 7, wherein the sensor (193) is a liquid level sensor, which comprises two electrodes (194) fixed to an inner wall of the communication tube (192) with a gap, and a control circuit board (195) electrically connected to the two electrodes (194) respectively, the control circuit board (195) is arranged in the main machine (2) and electrically connected to a CPU main control board (24);
when there is effusion at a position where the two electrodes (194) are located in the communication tube (192), the two electrodes (194) are conducted, and the control circuit board (195) transmits an electrical signal corresponding to the conduction of the two electrodes (194) to the CPU main control board (24); or, when there is no effusion at the position where the two electrodes (194) are located in the communication tube (192), the two electrodes (194) are disconnected, and the control circuit board (195) transmits an electrical signal corresponding to the disconnection of the two electrodes (194) to the CPU main control board (24).

9. The treatment pen (1) according to claim 1, wherein the vibrator (15) comprises a rotary centrifugal vibrator (150);
preferably, wherein, when the rotary centrifugal vibrator (150) that can be controlled by the switch button (14) to start and stop is arranged inside the pen rod (11), the treatment pen (1) further comprises a printed circuit board PCB control board (18) which is electrically connected to the rotary centrifugal vibrator (150) and used for outputting a vibration prescription signal to the rotary centrifugal vibrator (150), and a common mode filter is also integrated on the PCB control board (18).

10. A limb pain treatment instrument comprising the treatment pen (1) according to any one of claims 1 to 9 and a main machine (2),
wherein the main machine (2) comprises:
a chassis (21), which comprises a touch display screen (23) arranged on a surface of the chassis (21), and a central processing unit CPU main control board (24) arranged in the chassis (21) and electrically connected to the touch display screen (23), wherein the CPU main control board (24) is used for supplying power to the vibrator (15) and outputting the vibration prescription signal, and the touch display screen (23) is used for a user to select a vibration prescription, wherein the vibration prescription signal is output to the treatment pen (1).

11. The limb pain treatment instrument according to claim 10, wherein the chassis (21) comprises a base (211), a top bracket (212), and a side wall assembly (213) installed between the base (211) and the top bracket (212), wherein the base (211), the top bracket (212), and the side wall assembly (213) enclose a closed structure; at least three casters (214) are arranged at a bottom of the chassis (21);
preferably, wherein the top bracket (212) comprises two opposite upright plates (2121) and an arc-shaped plate (2122) located between the two upright plates (2121), and the touch display screen (23) is fixed on a surface of the arc-shaped plate (2122);
preferably, wherein the side wall assembly (213) comprises a rear cover plate (2131), a front plate (2132) arranged opposite to the rear cover plate (2131), and two side brackets (2133) arranged between the rear cover plate (2131) and the front plate (2132), a mounting plate (2134) is connected between the two side brackets (2133), the CPU main control board (24) is fixed on the mounting plate (2134), and a U-shaped handle (2135) is pivotally arranged on outer sides of the two side brackets (2133);
preferably, wherein opposite surfaces of the two side brackets (2133) are both provided with sliding grooves (2136), and the mounting plate (2134) is slidably connected in the sliding grooves (2136);
preferably, wherein the side bracket (2133) comprises an outer side plate (2137), a plurality of first rib plates (2138) fixedly connected to an inner side of the outer side plate (2137) and arranged along a height direction of the outer side plate (2137), and a plurality of second rib plates (2139) arranged along a width direction of the outer side plate (2137), wherein a sliding groove (2136) is formed between two adjacent second rib plates (2139).

12. The limb pain treatment instrument according to claim 11, wherein a receiving box (215) is arranged below the mounting plate (2134), and a bottom of the mounting plate 2134 is provided with an ultraviolet disinfection lamp 216, and the ultraviolet disinfection lamp 216 is used for ultraviolet disinfection of items placed in the receiving box 215.

13. The limb pain treatment instrument according to claim 10, wherein the main machine (2) further comprising at least one intermediate frequency output interface (26) arranged on the surface of the chassis (21);
the limb pain treatment instrument further comprises at least one group of electrode sheets (3) electrically connected to the at least one intermediate frequency output interface (26) via cables respectively, and the CPU main control board (24) is also electrically connected to the at least one intermediate frequency output interface (26) for outputting a current prescription signal to the at least one group of electrode sheets (3);
or
wherein characteristic parameters of the vibration prescription signal comprise: a continuous vibration of the vibrator (15) and a vibration frequency and an amplitude of the continuous vibration; or, an intermittent vibration of the vibrator (15) and a duty ratio, a vibration frequency and an amplitude of the intermittent vibration;
or
wherein a timer and a counter are integrated in the touch display screen (23), and the timer is used to turn off the main machine (2) or send an alarm when a duration for the main machine (2) outputting the vibration prescription signal to the treatment pen (1) reaches a preset duration threshold; the counter is used to record the number of times that the main machine (2) outputs the vibration prescription signal to the treatment pen (1).

14. The limb pain treatment instrument according to claim 10, wherein the vibration prescription comprises: a stored default fixed parameter of each treatment site and an individual vibration intensity level manually adjusted for a same treatment site;
the touch display screen (23) is used for the user to select a corresponding vibration prescription for a treatment site, and is used to generate target characteristic parameters together according to the default fixed parameter included in the selected vibration prescription and the individual vibration intensity level manually adjusted by the user for the same treatment site, and send the target characteristic parameters generated according to the vibration prescription selected by the user to the CPU main control board (24); the main CPU control board (24) is used to generate a corresponding vibration prescription signal according to the target characteristic parameters.

15. The limb pain treatment instrument according to claim 14, wherein the CPU main control board (24) comprises a microcomputer chip (241) and a triode (242);
the microcomputer chip (241) is used to receive the target characteristic parameters and determine a target duty ratio according to an amplitude in the target characteristic parameters and a pre-stored correspondence between amplitudes and duty ratios; generate the vibration prescription signal according to the target duty ratio and a vibration frequency in the target characteristic parameters, and send the vibration prescription signal to the triode (242);
the triode (242) is used to output the vibration prescription signal to the treatment pen (1) and controlling the vibration prescription signal to drive the vibrator (15).

16. The limb pain treatment instrument according to claim 10, wherein a display interface of the touch display screen (23) is provided with a vibration prescription selection button and a running button, wherein the vibration prescription selection button is used for a user to select a corresponding vibration prescription for a treatment site, and a CPU main control panel (24) of the main machine (2) can transmit a vibration prescription signal corresponding to the vibration prescription to the treatment pen (1) after a user clicks the running button, and the treatment pen (1) controls the vibrator (15) to vibrate according to the vibration prescription signal when the vibration prescription signal is received and the switch button (14) is turned on.

## Patentansprüche

1. Behandlungsstift (1) für ein Instrument zur Behandlung von Gliederschmerzen, umfassend eine Hauptmaschine (2), die ein Vibrationsvorgabesignal an den Behandlungsstift (1) ausgibt, wobei der Behandlungsstift (1) umfasst:
einen Stiftstab (11) und eine Stiftkappe (12), die abnehmbar mit einem distalen Ende des Stiftstabs (11) verbunden ist, eine Behandlungsnadel (13) ist in dem Stiftstab (11) angebracht, nachdem die Stiftkappe (12) abgenommen ist, eine Umschalttaste (14) ist an einer Oberfläche des Stiftstabs (11) bereitgestellt; ein Nadelrohr (131) der Behandlungsnadel (13) erstreckt sich aus einem distalen Ende der Stiftkappe (12), ein Vibrator (15), der durch die Umschalttaste(14) zum Starten und Stoppen gesteuert werden kann, ist innerhalb des Stiftstabs (11) bereitgestellt; der Vibrator (15) ist konfiguriert, um, wenn er gestartet wird, physikalische kinetische Energie entsprechend einem Vibrationsvorgabesignal an die in eine Läsionsstelle eingeführte Behandlungsnadel (13) zu senden,
**dadurch gekennzeichnet, dass** am distalen Ende des Stiftstabs (11) ein elastischer Wellenwulst (16) zum Anbringen und Lösen der Stiftkappe (12) bereitgestellt ist; eine Kontaktoberfläche (121) innerhalb der Stiftkappe (12) bereitgestellt ist, und die Kontaktoberfläche (121) sich am distalen Ende der Stiftkappe (12) befindet; und die Behandlungsnadel (13) auch eine Nadelrohrhalterung (132) umfasst, die fest mit dem Nadelrohr (131) verbunden ist, und wenn die Behandlungsnadel (13) an dem Stiftstab (11) befestigt ist, eine distale Endoberfläche der Nadelrohrhalterung (132) mit der Kontaktoberfläche (121) in Kontakt kommt.

2. Behandlungsstift (1) nach Anspruch 1, wobei die Nadelrohrhalterung (132) einen Nadelrohrbefestigungsteil (1321) und einen Klemmteil (1322) umfasst, der fest mit dem Nadelrohrbefestigungsteil (1321) verbunden ist, wobei der Nadelrohrbefestigungsteil (1321) mit einem Nadelrohrmontageloch (1323) versehen ist, das den Nadelrohrbefestigungsteil (1321) durchdringt, das Nadelrohr (131) in das Nadelrohrmontageloch (1323) eingesetzt und darin befestigt ist, der Klemmteil (1322) ringförmig und hohl ist, und ein Hohlraum des Klemmteils (1322) mit dem Nadelrohrmontageloch (1323) in Verbindung steht; das distale Ende der Stiftkappe (12) mit einer Nadelrohraustrittsöffnung (122) versehen ist, und das Nadelrohr (131) durch die Nadelrohraustrittsöffnung (122) passieren und sich aus dem distalen Ende der Stiftkappe (12) erstrecken kann;
der Behandlungsstift (1) auch eine Montagehalterung (17) umfasst, die innerhalb des Stiftstabs (11) bereitgestellt ist, wobei die Montagehalterung (17) einen Halterungskörper (171), der sich entlang einer Längenrichtung des Stiftstabs (11) erstreckt, und ein Mastoid (172) umfasst, das fest mit dem distalen Ende des Halterungskörpers (171) verbunden ist; und der Klemmteil (1322) mit einer Hülse versehen und auf dem Mastoid (172) befestigt ist;
vorzugsweise, wobei der Halterungskörper (171) einen ersten Körperteil (1711) und einen zweiten Körperteil (1712) umfasst, die gegenüberliegend angeordnet und fest verbunden sind, und der Vibrator (15) zwischen dem ersten Körperteil (1711) und dem zweiten Körperteil (1712) fest verbunden ist;
die Montagehalterung (17) ferner eine Seitenwand (173) umfasst, die an distalen Enden des ersten Körperteils (1711) und des zweiten Körperteils (1712) befestigt ist und das Mastoid (172) umgibt, und die Seitenwand (173) mit einer äußeren Seitenoberfläche der Nadelrohrhalterung (132) in Kontakt steht.

3. Behandlungsstift (1) nach Anspruch 1, wobei die Stiftkappe (12) mit mindestens einem Sichtfenster (123) entlang einer Umfangsrichtung versehen ist und die Nadelrohrhalterung (132) eine Nadelrohrhalterung aus transparentem Material ist und die Nadelrohrhalterung (132) durch jedes Sichtfenster (123) beobachtet werden kann.

4. Behandlungsstift (1) nach Anspruch 1, wobei eine Steuerplatine (18) einer gedruckten Leiterplatte PCB auch im Inneren des Stiftstabs (11) angeordnet ist, eine LED-Hintergrundbeleuchtung (181), eine Vibrationsanzeigelampe (182) und die Umschalttaste (14) auf der PCB-Steuerplatine (18) integriert sind, die LED-Hintergrundbeleuchtung (181) und der Schaltknopf (14) auf einer Oberfläche des Stiftstabs (11) freigelegt sind und ein Arbeitsbeobachtungsloch ebenfalls auf der Oberfläche des Stiftstabs (11) angeordnet ist, das Arbeitsbeobachtungsloch einer Position der Vibrationsanzeigelampe (182) entspricht und die LED-Hintergrundbeleuchtung (181) näher am distalen Ende des Stiftstabs (11) ist als die Vibrationsanzeigelampe (182), die PCB-Steuerplatine (18) auch elektrisch mit dem Vibrator (15) verbunden ist.

5. Behandlungsstift (1) nach Anspruch 1, wobei der Behandlungsstift (1) mindestens eine der folgenden Eigenschaften aufweist: (a) eine vom Vibrator (15) ausgegebene Vibrationsspur liegt in einer Ebene senkrecht oder parallel zu einer Achse des Stiftstabs (11), und
die Spur der Vibration umfasst eine Spur der Hin- und Herbewegung in einer einzigen Richtung und/oder eine Spur der Hin- und Herbewegung in mehreren Winkelrichtungen, die zyklisch um die Achse des Stiftstabs (11) zentriert sind; (b) eine Amplitude des Vibrators (15) ist zwischen 2 µm und 500 µm vorgesehen, und eine Vibrationsfrequenz des Vibrators (15) ist zwischen 5 Hz und 500 Hz vorgesehen; (c) die Behandlungsnadel (13) ist eine spezielle Behandlungsnadel oder eine Einwegspritzennadel, die verwendet wird, um in die durch eine Weichteilverletzung verursachte Läsionsstelle zur Diagnose und/oder Behandlung einzudringen.

6. Behandlungsstift (1) nach Anspruch 1, wobei der Behandlungsstift (1) ferner eine Unterdruckpumpe (19) umfasst, die abnehmbar mit dem distalen Ende des Stiftstabs (11) verbunden ist; die Stiftkappe (12) eine Stiftkappe aus transparentem Material ist; ein Gelenk (124) auf einer Oberfläche der Stiftkappe (12) befestigt ist; die Unterdruckpumpe (19) über ein Drainagerohr (191) mit dem Gelenk (124) verbunden ist; das Gelenk (124) mit der Stiftkappe (12) und der Behandlungsnadel (13) in Verbindung steht; und die Unterdruckpumpe (19) zum Bereitstellen eines Saugunterdrucks für den Erguss innerhalb der Läsionsstelle beim Starten verwendet wird, sodass der Erguss von der Läsionsstelle abgepumpt werden kann.

7. Behandlungsstift (1) nach Anspruch 6, wobei der Behandlungsstift (1) ferner ein Verbindungsrohr (192) umfasst, welches das Drainagerohr (191) und das Gelenk (124) verbindet; ein Steuersignal zum Starten und Stoppen der Unterdruckpumpe (19) von der Hauptmaschine (2) kommt, und die Hauptmaschine (2) ein entsprechendes Steuersignal erzeugt, indem sie erkennt, ob an einer Position, an der sich ein Sensor (193) im Verbindungsrohr (192) befindet, ein Erguss vorliegt.

8. Behandlungsstift (1) nach Anspruch 7, wobei der Sensor (193) ein Flüssigkeitspegelsensor ist, der zwei Elektroden (194) umfasst, die mit einem Spalt an einer Innenwand des Verbindungsrohrs (192) befestigt sind, und eine Steuerplatine (195), die jeweils mit den beiden Elektroden (194) elektrisch verbunden ist, wobei die Steuerplatine (195) in der Hauptmaschine (2) angeordnet und elektrisch mit einer CPU-Hauptsteuerplatine (24) verbunden ist;
wenn ein Erguss an einer Position vorliegt, an der sich die beiden Elektroden (194) in dem Verbindungsrohr (192) befinden, die beiden Elektroden (194) geleitet werden, und die Steuerplatine (195) ein elektrisches Signal, das der Leitung der beiden Elektroden (194) entspricht, an die CPU-Hauptsteuerplatine (24) sendet; oder, wenn an der Position, an der sich die beiden Elektroden (194) in dem Verbindungsrohr (192) befinden, kein Erguss vorliegt, die beiden Elektroden (194) getrennt werden und die Steuerplatine (195) ein elektrisches Signal, das der Trennung der beiden Elektroden (194) entspricht, an die CPU-Hauptsteuerplatine (24) sendet.

9. Behandlungsstift (1) nach Anspruch 1, wobei der Vibrator (15) einen Rotationszentrifugalvibrator (150) umfasst;
vorzugsweise, wobei, wenn der Rotationszentrifugalvibrator (150), der durch die Umschalttaste (14) zum Starten und Stoppen gesteuert werden kann, innerhalb des Stiftstabs (11) angeordnet ist, der Behandlungsstift (1) ferner eine Steuerplatine (18) einer gedruckten Leiterplatte PCB umfasst, die elektrisch mit dem Rotationszentrifugalvibrator (150) verbunden ist und zur Ausgabe eines Vibrationsvorgabesignals an den Rotationszentrifugalvibrator (150) verwendet wird, und ein Gleichtaktfilter ebenfalls auf der PCB-Steuerplatine (18) integriert ist.

10. Instrument zur Behandlung von Gliederschmerzen, das den Behandlungsstift (1) nach einem der Ansprüche 1 bis 9 und eine Hauptmaschine (2) umfasst,
wobei die Hauptmaschine (2) umfasst:
ein Gestell (21), das einen auf einer Oberfläche des Gestells (21) angeordneten Berührungsanzeigebildschirm (23) umfasst, und eine in dem Gestell (21) angeordnete und mit dem Berührungsanzeigebildschirm (23) elektrisch verbundene CPU-Hauptsteuerplatine (24) einer zentralen Verarbeitungseinheit, wobei die CPU-Hauptsteuerplatine (24) zur Energieversorgung des Vibrators (15) und zur Ausgabe des Vibrationsvorgabesignals dient, und der Berührungsanzeigebildschirm (23) zur Auswahl einer Vibrationsvorgabe durch einen Benutzer dient, wobei das Vibrationsvorgabesignal an den Behandlungsstift (1) ausgegeben wird.

11. Instrument zur Behandlung von Gliederschmerzen nach Anspruch 10, wobei das Gestell (21) eine Basis (211), eine obere Halterung (212) und eine zwischen der Basis (211) und der oberen Halterung (212) eingebaute Seitenwandanordnung (213) umfasst, wobei die Basis (211), die obere Halterung (212) und die Seitenwandanordnung (213) eine geschlossene Struktur umschließen; mindestens drei Rollen (214) an einem Boden des Gestells (21) angeordnet sind;
vorzugsweise, wobei die obere Halterung (212) zwei gegenüberliegende aufrechte Platten (2121) und eine bogenförmige Platte (2122) umfasst, die sich zwischen den beiden aufrechten Platten (2121) befindet, und der Berührungsanzeigebildschirm (23) auf einer Oberfläche der bogenförmigen Platte (2122) befestigt ist;
vorzugsweise, wobei die Seitenwandanordnung (213) eine hintere Abdeckplatte (2131), eine gegenüber der hinteren Abdeckplatte (2131) angeordnete vordere Abdeckplatte (2132) und zwei zwischen der hinteren Abdeckplatte (2131) und der vorderen Abdeckplatte (2132) angeordnete Seitenhalterungen (2133) umfasst, eine Montageplatte (2134) zwischen den beiden Seitenhalterungen (2133) verbunden ist, die CPU-Hauptsteuerplatine (24) an der Montageplatte (2134) befestigt ist, und ein U-förmiger Griff (2135) schwenkbar auf Außenseiten der beiden Seitenhalterungen (2133) angeordnet ist;
vorzugsweise, wobei gegenüberliegende Oberflächen der beiden Seitenhalterungen (2133) beide mit Gleitnuten (2136) versehen sind und die Montageplatte (2134) in den Gleitnuten (2136) gleitbar verbunden ist;
vorzugsweise, wobei die Seitenhalterung (2133) eine äußere Seitenplatte (2137), eine Vielzahl von ersten Rippenplatten (2138), die fest mit einer Innenseite der äußeren Seitenplatte (2137) verbunden und entlang einer Höhenrichtung der äußeren Seitenplatte (2137) angeordnet sind, und eine Vielzahl von zweiten Rippenplatten (2139), die entlang einer Breitenrichtung der äußeren Seitenplatte (2137) angeordnet sind, umfasst, wobei eine Gleitnut (2136) zwischen zwei aneinandergrenzenden zweiten Rippenplatten (2139) ausgebildet ist.

12. Instrument zur Behandlung von Gliederschmerzen nach Anspruch 11, wobei ein Aufnahmekasten (215) unterhalb der Montageplatte (2134) angeordnet ist und ein Boden der Montageplatte 2134 mit einer Ultraviolett-Desinfektionslampe 216 versehen ist und die Ultraviolett-Desinfektionslampe 216 zur Ultraviolett-Desinfektion von in den Aufnahmekasten 215 platzierten Gegenständen verwendet wird.

13. Instrument zur Behandlung von Gliederschmerzen nach Anspruch 10, wobei die Hauptmaschine (2) ferner mindestens eine Zwischenfrequenz-Ausgangsschnittstelle (26) umfasst, die auf der Oberfläche des Gestells (21) angeordnet ist;
das Instrument zur Behandlung von Gliederschmerzen ferner mindestens eine Gruppe von Elektrodenblättern (3) umfasst, die jeweils über Kabel elektrisch mit der mindestens einen Zwischenfrequenz-Ausgangsschnittstelle (26) verbunden sind, und die CPU-Hauptsteuerplatine (24) ebenfalls elektrisch mit der mindestens einen Zwischenfrequenz-Ausgangsschnittstelle (26) verbunden ist, um ein Stromvorgabesignal an die mindestens eine Gruppe von Elektrodenblättern (3) auszugeben;
oder
wobei charakteristische Parameter des Vibrationsvorgabesignals folgendes umfassen: eine kontinuierliche Vibration des Vibrators (15) und eine Vibrationsfrequenz und eine Amplitude der kontinuierlichen Vibration; oder eine intermittierende Vibration des Vibrators (15) und ein Tastverhältnis, eine Vibrationsfrequenz und eine Amplitude der intermittierenden Vibration;
oder
wobei ein Zeitgeber und ein Zähler in den Berührungsanzeigebildschirm (23) integriert sind und der Zeitgeber verwendet wird, um die Hauptmaschine (2) auszuschalten oder einen Alarm zu senden, wenn eine Dauer für die Hauptmaschine (2), die das Vibrationsvorgabesignal an den Behandlungsstift (1) ausgibt, einen voreingestellten Dauerschwellenwert erreicht; der Zähler dient dazu, die Anzahl der Male zu erfassen, die die Hauptmaschine (2) das Vibrationsvorgabesignal an den Behandlungsstift (1) ausgibt.

14. Instrument zur Behandlung von Gliederschmerzen nach Anspruch 10, wobei die Vibrationsvorgabe umfasst: einen gespeicherten festen Standardparameter für jede Behandlungsstelle und ein individuelles Vibrationsintensitätsniveau, das manuell für dieselbe Behandlungsstelle angepasst wird;
der Berührungsanzeigebildschirm (23) für den Benutzer verwendet wird, um eine entsprechende Vibrationsvorgabe für eine Behandlungsstelle auszuwählen, und verwendet wird, um Zielcharakteristikparameter zusammen gemäß dem festen Standardparameter, der in der ausgewählten Vibrationsvorgabe eingeschlossen ist, und dem individuellen Vibrationsintensitätsniveau, das vom Benutzer für dieselbe Behandlungsstelle manuell gesteuert wird, zu erzeugen, und die Zielcharakteristikparameter, die gemäß der vom Benutzer ausgewählten Vibrationsvorgabe erzeugt werden, an die CPU-Hauptsteuerplatine (24) zu senden; die CPU-Hauptsteuerplatine (24) verwendet wird, um ein entsprechendes Vibrationsvorgabesignal gemäß den Zielcharakteristikparametern zu erzeugen.

15. Instrument zur Behandlung von Gliederschmerzen nach Anspruch 14, wobei die CPU-Hauptsteuerplatine (24) einen Mikrocomputerchip (241) und eine Triode (242) umfasst;
der Mikrocomputerchip (241) verwendet wird, um die Zielcharakteristikparameter zu empfangen und ein Zieltastverhältnis entsprechend einer Amplitude in den Zielcharakteristikparametern und einer vorgespeicherten Korrespondenz zwischen Amplituden und Tastverhältnissen zu bestimmen; Erzeugen des Vibrationsvorgabesignals entsprechend dem Zieltastverhältnis und einer Vibrationsfrequenz in den Zielcharakteristikparametern, und Senden des Vibrationsvorgabesignals an die Triode (242);
die Triode (242) zur Ausgabe des Vibrationsvorgabesignals an den Behandlungsstift (1) und zur Steuerung des Vibrationsvorgabesignals zur Ansteuerung des Vibrators (15) verwendet wird.

16. Instrument zur Behandlung von Gliederschmerzen nach Anspruch 10, wobei eine Anzeigeschnittstelle des Berührungsanzeigebildschirms (23) mit einer Vibrationsvorgabeauswahltaste und einer Betriebstaste versehen ist, wobei die Vibrationsvorgabeauswahltaste für einen Benutzer verwendet wird, um eine entsprechende Vibrationsvorgabe für eine Behandlungsstelle auszuwählen, und eine CPU Hauptsteuertafel (24) der Hauptmaschine (2) ein Vibrationsvorgabesignal, das der Vibrationsvorgabe entspricht, an den Behandlungsstift (1) senden kann, nachdem ein Benutzer die Betriebstaste angeklickt hat, und der Behandlungsstift (1) den Vibrator (15) steuert, um gemäß dem Vibrationsvorgabesignal zu vibrieren, wenn das Vibrationsvorgabesignal empfangen wird und die Umschalttaste (14) eingeschaltet ist.

## Revendications

1. Stylo de traitement (1) pour un instrument de traitement de la douleur de membres comprenant une machine principale (2) qui émet un signal de prescription de vibration vers le stylo de traitement (1), dans lequel le stylo de traitement (1) comprend :
une tige de stylo (11) et un capuchon de stylo (12) relié de manière amovible à une extrémité distale de la tige de stylo (11), une aiguille de traitement (13) est montée dans la tige de stylo (11) après que le capuchon de stylo (12) a été détaché, un bouton interrupteur (14) est fourni sur une surface de la tige de stylo (11) ; un tube d'aiguille (131) de l'aiguille de traitement (13) s'étend hors d'une extrémité distale du capuchon de stylo (12), un vibrateur (15) dont la mise en marche et l'arrêt peuvent être commandés par le bouton interrupteur (14) est fourni à l'intérieur de la tige de stylo (11) ; le vibrateur (15) est configuré pour, lorsqu'il est mis en marche, transmettre une énergie cinétique physique correspondant à un signal de prescription de vibration à l'aiguille de traitement (13) introduite dans un site de lésion,
**caractérisé en ce qu'**un bourrelet élastique (16) permettant de monter et de démonter le capuchon de stylo (12) est fourni au niveau de l'extrémité distale de la tige de stylo (11) ; une surface de contact (121) est prévue à l'intérieur du capuchon de stylo (12), et la surface de contact (121) est située au niveau de l'extrémité distale du capuchon de stylo (12) ; et l'aiguille de traitement (13) comprend également un support de tube d'aiguille (132) relié de manière fixe au tube d'aiguille (131), et lorsque l'aiguille de traitement (13) est fixée sur la tige de stylo (11), une surface d'extrémité distale du support de tube d'aiguille (132) entre en contact avec la surface de contact (121).

2. Stylo de traitement (1) selon la revendication 1, dans lequel le support de tube d'aiguille (132) comprend une partie de fixation de tube d'aiguille (1321) et une partie de serrage (1322) reliée de manière fixe à la partie de fixation de tube d'aiguille (1321), dans lequel la partie de fixation de tube d'aiguille (1321) est pourvue d'un trou de montage de tube d'aiguille (1323) pénétrant à travers la partie de fixation de tube d'aiguille (1321), le tube d'aiguille (131) est inséré et fixé à l'intérieur du trou de montage de tube d'aiguille (1323), la partie de serrage (1322) est annulaire et creuse, et une cavité de la partie de serrage (1322) est en communication avec le trou de montage de tube d'aiguille (1323) ; l'extrémité distale du capuchon de stylo (12) est pourvue d'un trou de sortie de tube d'aiguille (122), et le tube d'aiguille (131) peut passer à travers le trou de sortie de tube d'aiguille (122) et s'étendre hors de l'extrémité distale du capuchon de stylo (12) ;
le stylo de traitement (1) comprend également un support de montage (17) fourni à l'intérieur de la tige de stylo (11), dans lequel le support de montage (17) comprend un corps de support (171) s'étendant dans un sens de la longueur de la tige de stylo (11) et une mastoïde (172) reliée de manière fixe à l'extrémité distale du corps de support (171) ; et la partie de serrage (1322) est manchonnée et fixée sur la mastoïde (172) ;
de préférence, dans lequel le corps de support (171) comprend une première partie de corps (1711) et une seconde partie de corps (1712) qui sont agencées de manière opposée et reliées de manière fixe, et le vibrateur (15) est relié de manière fixe entre la première partie de corps (1711) et la seconde partie de corps (1712) ;
le support de montage (17) comprend en outre une paroi latérale (173) fixée aux extrémités distales de la première partie de corps (1711) et de la seconde partie de corps (1712) et entourant la mastoïde (172), et la paroi latérale (173) est en contact avec une surface latérale extérieure du support de tube d'aiguille (132).

3. Stylo de traitement (1) selon la revendication 1, dans lequel le capuchon de stylo (12) est pourvu d'au moins une fenêtre d'observation (123) le long d'une direction circonférentielle, et le support de tube d'aiguille (132) est un support de tube d aiguille fait d'un matériau transparent, et le support de tube d'aiguille (132) peut être observé à travers chaque fenêtre d'observation (123).

4. Stylo de traitement (1) selon la revendication 1, dans lequel un tableau de commande de carte de circuit imprimé, PCB (18) est également agencé à l'intérieur de la tige de stylo (11), un rétroéclairage à DEL (181), un témoin de vibration (182) et le bouton interrupteur (14) sont intégrés sur le tableau de commande de PCB (18), le rétroéclairage à DEL (181) et le bouton interrupteur (14) sont exposés sur une surface de la tige de stylo (11), et un trou d'observation de travail est également agencé sur la surface de la tige de stylo (11), le trou d'observation de travail correspond à une position du témoin de vibration (182), et le rétroéclairage à DEL (181) est plus proche de l'extrémité distale de la tige de stylo (11) que le témoin de vibration (182), le tableau de commande de PCB (18) est également connecté électriquement au vibrateur (15).

5. Stylo de traitement (1) selon la revendication 1, dans lequel le stylo de traitement (1) a au moins l'une des caractéristiques suivantes : (a) une trace de vibration émise par le vibrateur (15) se trouve dans un plan perpendiculaire ou parallèle à un axe de la tige de stylo (11), et
la trace de vibration comprend une trace de mouvement alternatif dans une seule direction, et/ou une trace de mouvement alternatif dans plusieurs directions angulaires cycliquement centrées sur l'axe de la tige de stylo (11) ; (b) une amplitude du vibrateur (15) est conçue entre 2 µm et 500 µm, et une fréquence de vibration du vibrateur (15) est conçue entre 5 Hz et 500 Hz ; (c) l'aiguille de traitement (13) est une aiguille de traitement spécialisée ou une aiguille de seringue jetable, qui est utilisée pour intervenir sur le site de la lésion provoquée par un dommage aux tissus mous à des fins de diagnostic et/ou de traitement.

6. Stylo de traitement (1) selon la revendication 1, dans lequel le stylo de traitement (1) comprend en outre une pompe à pression négative (19) reliée de manière amovible à l'extrémité distale de la tige de stylo (11) ; le capuchon de stylo (12) est un capuchon de stylo en matériau transparent ; un joint (124) est fixé sur une surface du capuchon de stylo (12) ; la pompe à pression négative (19) est reliée au joint (124) à travers un tube de drainage (191) ; le joint (124) est en communication avec le capuchon de stylo (12) et l'aiguille de traitement (13) ; et la pompe à pression négative (19) est utilisée pour fournir une pression négative d'aspiration pour l'épanchement à l'intérieur du site de la lésion lorsqu'elle est mise en marche, de sorte que l'épanchement puisse être pompé hors du site de la lésion.

7. Stylo de traitement (1) selon la revendication 6, dans lequel le stylo de traitement (1) comprend en outre un tube de communication (192) reliant le tube de drainage (191) et le joint (124) ; un signal de commande pour la mise en marche et l'arrêt de la pompe à pression négative (19) provient de la machine principale (2), et la machine principale (2) génère un signal de commande correspondant en détectant s'il y a un épanchement au niveau d'une position où se trouve un capteur (193) dans le tube de communication (192).

8. Stylo de traitement (1) selon la revendication 7, dans lequel le capteur (193) est un capteur de niveau de liquide, qui comprend deux électrodes (194) fixées à une paroi intérieure du tube de communication (192) avec un espace, et une carte de circuit de commande (195) connectée électriquement aux deux électrodes (194) respectivement, la carte de circuit de commande (195) est agencée dans la machine principale (2) et connectée électriquement à une carte de commande principale de l'unité centrale de traitement, CPU (24) ;
lorsqu'il y a un épanchement au niveau d'une position où les deux électrodes (194) sont situées dans le tube de communication (192), les deux électrodes (194) sont conduites, et la carte de circuit de commande (195) transmet un signal électrique correspondant à la conduction des deux électrodes (194) à la carte de commande principale de l'CPU (24) ; ou, lorsqu'il n'y a pas d'épanchement au niveau de la position où les deux électrodes (194) sont situées dans le tube de communication (192), les deux électrodes (194) sont déconnectées, et la carte de circuit de commande (195) transmet un signal électrique correspondant à la déconnexion des deux électrodes (194) à la carte de commande principale de l'CPU (24).

9. Stylo de traitement (1) selon la revendication 1, dans lequel le vibrateur (15) comprend un vibrateur centrifuge rotatif (150) ;
de préférence, dans lequel, lorsque le vibrateur centrifuge rotatif (150), dont la mise en marche et l'arrêt peuvent être commandés par le bouton interrupteur (14), est agencé à l'intérieur de la tige de stylo (11), le stylo de traitement (1) comprend en outre un tableau de commande de carte de circuit imprimé PCB (18) qui est connecté électriquement au vibrateur centrifuge rotatif (150) et utilisé pour émettre un signal de prescription de vibration vers le vibrateur centrifuge rotatif (150), et un filtre de mode commun est également intégré au tableau de commande de PCB (18).

10. Instrument de traitement de la douleur de membres comprenant le stylo de traitement (1) selon l'une quelconque des revendications 1 à 9 et une machine principale (2),
dans lequel la machine principale (2) comprend :
un châssis (21), qui comprend un écran d'affichage tactile (23) agencé sur une surface du châssis (21), et une carte de commande principale de l'unité centrale de traitement, CPU (24) agencée dans le châssis (21) et connectée électriquement à l'écran d'affichage tactile (23), dans lequel la carte de commande principale de l'CPU (24) est utilisée pour alimenter le vibrateur (15) et émettre le signal de prescription de vibration, et l'écran d'affichage tactile (23) est utilisé pour qu'un utilisateur sélectionne une prescription de vibration, dans lequel le signal de prescription de vibration est émis vers le stylo de traitement (1).

11. Instrument de traitement de la douleur de membres selon la revendication 10, dans lequel le châssis (21) comprend une base (211), un support supérieur (212) et un ensemble paroi latérale (213) installé entre la base (211) et le support supérieur (212), dans lequel la base (211), le support supérieur (212) et l'ensemble paroi latérale (213) entourent une structure fermée ; au moins trois roulettes (214) sont agencées au niveau d'une partie inférieure du châssis (21) ;
de préférence, dans lequel le support supérieur (212) comprend deux plaques verticales opposées (2121) et une plaque en forme d'arc (2122) située entre les deux plaques verticales (2121), et l'écran d'affichage tactile (23) est fixé sur une surface de la plaque en forme d'arc (2122) ;
de préférence, dans lequel l'ensemble paroi latérale (213) comprend une plaque de recouvrement arrière (2131), une plaque avant (2132) agencée à l'opposé de la plaque de recouvrement arrière (2131), et deux supports latéraux (2133) agencés entre la plaque de recouvrement arrière (2131) et la plaque avant (2132), une plaque de montage (2134) est reliée entre les deux supports latéraux (2133), la carte de commande principale de l'CPU (24) est fixée sur la plaque de montage (2134), et une poignée en forme de U (2135) est agencée de manière pivotante sur les côtés extérieurs des deux supports latéraux (2133) ;
de préférence, dans lequel les surfaces opposées des deux supports latéraux (2133) sont toutes deux pourvues de rainures de glissement (2136), et la plaque de montage (2134) est reliée de manière coulissante dans les rainures de glissement (2136) ;
de préférence, dans lequel le support latéral (2133) comprend une plaque latérale extérieure (2137), une pluralité de premières plaques nervurées (2138) reliées de manière fixe à un côté intérieur de la plaque latérale extérieure (2137) et agencées dans un sens de la hauteur de la plaque latérale extérieure (2137), et une pluralité de secondes plaques nervurées (2139) agencées dans un sens de la largeur de la plaque latérale extérieure (2137), dans lequel une rainure de glissement (2136) est formée entre deux secondes plaques nervurées adjacentes (2139).

12. Instrument de traitement de la douleur de membres selon la revendication 11, dans lequel une boîte de réception (215) est agencée sous la plaque de montage (2134), et une partie inférieure de la plaque de montage 2134 est pourvue d'une lampe de désinfection aux ultraviolets 216, et la lampe de désinfection aux ultraviolets 216 est utilisée pour la désinfection aux ultraviolets des articles placés dans la boîte de réception 215.

13. Instrument de traitement de la douleur de membres selon la revendication 10, dans lequel la machine principale (2) comprend en outre au moins une interface de sortie de fréquence intermédiaire (26) agencée sur la surface du châssis (21) ;
l'instrument de traitement de la douleur de membres comprend en outre au moins un groupe de feuilles d'électrodes (3) connectées électriquement à l'au moins une interface de sortie de fréquence intermédiaire (26) par l'intermédiaire de câbles respectivement, et la carte de commande principale de l'CPU (24) est également connectée électriquement à l'au moins une interface de sortie de fréquence intermédiaire (26) pour émettre un signal de prescription de courant vers l'au moins un groupe de feuilles d'électrodes (3) ;
ou
dans lequel les paramètres caractéristiques du signal de prescription de vibration comprennent : une vibration continue du vibrateur (15) et une fréquence de vibration ainsi qu'une amplitude de la vibration continue ; ou, une vibration intermittente du vibrateur (15) et un rapport d'utilisation, une fréquence de vibration et une amplitude de la vibration intermittente ;
ou
dans lequel une minuterie et un compteur sont intégrés à l'écran d'affichage tactile (23), et la minuterie est utilisée pour éteindre la machine principale (2) ou envoyer une alarme lorsqu'une durée pendant laquelle la machine principale (2) émet le signal de prescription de vibration vers le stylo de traitement (1) atteint un seuil de durée prédéfini ; le compteur est utilisé pour enregistrer le nombre de fois que la machine principale (2) envoie le signal de prescription de vibration au stylo de traitement (1).

14. Instrument de traitement de la douleur de membres selon la revendication 10, dans lequel la prescription de vibration comprend : un paramètre fixe par défaut stocké pour chaque site de traitement et un niveau d'intensité de vibration individuel ajusté manuellement pour un même site de traitement ;
l'écran d'affichage tactile (23) permet à l'utilisateur de sélectionner une prescription de vibration correspondante pour un site de traitement et de générer des paramètres caractéristiques cibles selon le paramètre fixe par défaut inclus dans la prescription de vibration sélectionnée et du niveau d'intensité de vibration individuel ajusté manuellement par l'utilisateur pour le même site de traitement, et d'envoyer les paramètres caractéristiques cibles générés selon la prescription de vibration sélectionnée par l'utilisateur à la carte de commande principale de l'CPU (24) ; la carte de commande principale de l'CPU (24) est utilisée pour générer un signal de prescription de vibration correspondant selon des paramètres caractéristiques cibles.

15. Instrument de traitement de la douleur de membres selon la revendication 14, dans lequel la carte de commande principale de l'CPU (24) comprend une puce de micro-ordinateur (241) et une triode (242) ;
la puce de micro-ordinateur (241) est utilisée pour recevoir les paramètres caractéristiques cibles et déterminer un rapport d'utilisation cible selon une amplitude dans les paramètres caractéristiques cibles et une correspondance préenregistrée entre les amplitudes et les rapports d'utilisation ; générer le signal de prescription de vibration selon le rapport d'utilisation cible et une fréquence de vibration dans les paramètres caractéristiques cibles, et envoyer le signal de prescription de vibration à la triode (242) ;
la triode (242) est utilisée pour émettre le signal de prescription de vibration vers le stylo de traitement (1) et pour commander le signal de prescription de vibration afin d'entraîner le vibrateur (15).

16. Instrument de traitement de la douleur de membres selon la revendication 10, dans lequel une interface d'affichage de l'écran d'affichage tactile (23) est pourvue d'un bouton de sélection de prescription de vibration et d'un bouton de marche, dans lequel le bouton de sélection de prescription de vibration est utilisé par un utilisateur pour sélectionner une prescription de vibration correspondante pour un site de traitement, et un panneau de commande principal de l'CPU (24) de la machine principale (2) peut transmettre un signal de prescription de vibration correspondant à la prescription de vibration au stylo de traitement (1) après qu'un utilisateur a cliqué sur le bouton de marche, et le stylo de traitement (1) commande le vibrateur (15) pour qu'il vibre selon le signal de prescription de vibration lorsque le signal de prescription de vibration est reçu et que le bouton interrupteur (14) est allumé.
